# EUROPEAN PATENT SPECIFICATION

(11) **EP 3 459 512 B1**
(45) Date of publication and mention of the grant of the patent: **12.04.2023**
(21) Application number: 18735938.5
(22) Date of filing: 04.01.2018
(51) Int. Cl.: A61F 13/49, A61F 13/51, A61F 13/15

(54) **ABSORBENT ARTICLE**
SAUGFÄHIGER ARTIKEL
ARTICLE ABSORBANT

(30) Priority: 07.01.2017 JP 2017001488
(43) Date of publication of application: 27.03.2019
(73) Proprietor: Koyo Corporation, Kanagawa 236-0004 (JP)
(72) Inventor: SHIRAI Atsuko, Yokohama-shi Kanagawa 236-0004 (JP); MATSUMIYA Munetada, Yokohama-shi Kanagawa 236-0004 (JP)
(74) Representative: Samson & Partner Patentanwälte mbB
(86) International application number: PCT/JP2018/000035
(87) International publication number: WO 2018/128178

(56) References cited:
- WO-A1-2012/036599
- WO-A1-2016/121976
- WO-A1-2016/121982
- JP-A- 2015 204 982
- JP-A- 2016 067 627
- JP-A- 2016 189 931
- JP-B1- 6 049 228
- US-A1- 2008 095 978

## Description

### TECHNICAL FIELD

The present invention relates to an absorbent article including a disposable underwear such as an underpants-type disposable diaper using a laminated sheet in which an elastic film is layered between non-stretchable sheets such as nonwoven fabrics.

### BACKGROUND ART

Absorbent articles including disposable undergarments such as disposable diapers have various forms such as underpants-type, tape fastening type, pad type and the like. Among them, the underpants-type absorbent article is constituted by a ventral member, a crotch member and a dorsal member, and both ends of the left-right direction of the ventral member and the dorsal member are joined to form a waist opening and leg openings. The crotch member is provided with an absorber that absorbs body fluids.

This underpants-type absorbent article expands and contracts in a waistline direction and leg circumferential directions to be in close contact with a skin of a user to prevent slipping and leakage of body fluids and the like.

In general, this underpants-type absorbent article is given a elasticity in the waistline direction by providing a plurality of elastic members such as elastic strands to the ventral member and the dorsal member and is also given a elasticity in the leg-circumferential directions by providing a plurality of elastic members on leg-around portions of the ventral member, the crotch member and the dorsal member.

On the other hand, in order to improve the appearance of the underpants-type absorbent article using the elastic members such as elastic strands and its fitness to the user's skin, an underpants-type absorbent article using an elastic film instead of the elastic member such as elastic strands has also appeared.

In this underpants-type absorbent article using the elastic film, mainly a ventral member and a dorsal member are composed of a laminated sheet in which an elastic film is layered between non-stretchable sheets such as nonwoven fabric.

When forming this laminated sheet, an elongated elastic film is layered between non-stretchable sheets, heat sealing or ultrasonic sealing is performed in a number of dot-like patterns or linear patterns, and each layer of the layered sheet is joined. When each layer of the layered sheet is joined, the periphery of joining portions (sealed portions) of the elastic film is broken to form opening portions (through holes). In the laminated sheet, the non-stretchable sheets in which the elastic film is layered are joined to each other by the joining portion in the opening portion formed in the elastic film. The opening portion also functions to give air permeability to the absorbent article using the elastic film.

The shape, size, arrangement pattern and the like of the joining portion of the laminated sheet affect the elasticity, appearance, air permeability and the like of the absorbent article using the elastic film, so that various shapes, sizes, arrangement patterns and the like of the joining portion have been proposed.

For example, Patent Document 1(Japanese Patent No.5,980,355) discloses a stretchable structure of an absorbent article and an underpants-type disposable diaper using the same for the purpose of preventing an elastic film from breaking at the boundary between a stretchable region and a non-stretchable region. The elastic film is layered between a first sheet layer and a second sheet layer over the stretchable region and the non-stretchable region continuing from the stretchable region. With the elastic film stretched in the stretchable direction along its surface, the first sheet layer and the second sheet layer are joined to each other at a large number of dot-like joining portions arrayed at intervals in the stretchable direction and a direction perpendicular to the stretchable direction, via through holes formed in the elastic film. An end portion of the stretchable region adjacent to the non-stretchable region is a buffer stretchable section in which the area rate of the dot-like joining portions is lower than a main stretchable section excluding the end portion.

Patent Document 2 (Japanese Patent No. 5967736) discloses an absorbent article for the purpose of obtaining a stretchable region with more cloth-like appearance while suppressing a decrease in softness. The stretchable region is formed by laminating an elastic film between a first sheet layer made of a nonwoven fabric and a second sheet layer made of a nonwoven fabric. With the elastic film stretched in the stretchable direction of the stretchable region, the first sheet layer and the second sheet layer are joined to each other at only a large number of joining portions which are arranged at intervals in a staggered manner with reference to the stretchable direction and a direction perpendicular thereto, via through holes formed in the elastic film. The joining portions are elongated in the direction perpendicular to the stretchable direction and are shaped to be line symmetrical with respect to a center line passing through the center of the stretchable direction. The maximum width of the joining portions in the stretchable direction is 0.1 to 1.1 mm, and the interval of the joining portions aligned in the stretchable direction is 3 to 12.9 mm, and the interval between the joining portions aligned in the direction perpendicular to the stretchable direction is 2 to 10.5 mm.

FIG. 22A is an enlarged front view showing a part of the stretchable region of the outer member in the attached state of the absorbent article of Patent Document 1, and FIG. 22B is an enlarged front view showing a part of the stretchable region of the outer member in the attached state of the absorbent article of Patent Document 2.

In the figure, reference numerals 100 and 110 denote outer sheets formed by laminating an elastic film between a first sheet layer and a second sheet layer which are made of a nonwoven fabric or the like. 101 and 111 denote joining portions between a first sheet layer and a second sheet layer. 102 and 112 denote opening portions formed in the elastic film, that is through holes of Patent Documents 1 and 2.

As shown in FIG. 22A, in the stretchable region of the outer sheet 100 of the absorbent article of Patent Document 1, the first sheet layer and the second sheet layer are joined at a dot-like joining portion 101, and this joining portion 101 forms an opening portion 102 in an elastic film.

As shown in FIG. 22B, in the stretchable region of the outer sheet 110 of the absorbent article of Patent Document 2, the first sheet layer and the second sheet layer are joined by a longitudinally elongated linear joining portion 111, and this joining portion 111 forms an opening portion 112 in an elastic film.

When the absorbent article is worn by the user, the outer sheets 100 and 110 are elongated in the left-right direction(the waistline direction), and the opening portions 102 and 112 are elongated in the left-right direction to form elliptical shapes as shown by broken lines in FIG. 22A and FIG. 22B, and the opening portions 102 and 112 except for the joining portions 101 and 111 function as ventilation holes.

As shown in FIG. 22A and FIG. 22B, the joining portions 101 and 111 are arranged at intervals in a staggered manner.

By the way, the absorbent article using such an elastic film is required to reduce an extension stress (a tensile stress for generating a predetermined elongation (strain) to some extent to make the outer sheets 100 and 110 easier to stretch even with a small force, to increase a maximum elongation rate of elastic deformation of the outer sheets 100 and 110 (hereinafter referred to as "maximum elongation") more than a predetermined value, for example, the maximum elongation 200% or more with an elongation of natural length set as 100%, and to improve air permeability.

In the case where the joining portions 101 and 111 are arranged at intervals, if the interval between the joining portions 101 and 111 is narrowed or the joining portions 101 and 111 are enlarged, the outer sheet 100 and 110 have a reduced extension stress and are easy to stretch, and the interval between the opening portions 102 and 112 is narrowed or the opening portions 102 and 112 are enlarged, so that the air permeability is improved. However, there is a limit to increasing the maximum elongation of the outer sheets 100 and 110, and there is a problem that the maximum elongation of the outer sheets 100 and 110 cannot be increased beyond a predetermined value.

In addition, as described in Patent Document 2, in the case where the vertically elongated joining portions are arranged at intervals in the staggered manner with reference to the stretchable direction and the direction perpendicular thereto, since a region without the joining portion 111 (and the opening portion 112) continues in the direction perpendicular to the stretchable direction (the longitudinal direction of the joining portion), a continuous large wrinkle is formed there, and this wrinkle causes stiffness in the direction perpendicular to the stretchable direction so as to impair a softness of the absorbent article.
WO 2016/121976 A1 relates to an absorbent article comprising an elastic area formed by a first sheet layer made from nonwoven cloth and a second sheet layer made from nonwoven cloth, having laminated therebetween an elastic film. The first sheet layer and second sheet layer are joined via through holes formed in the elastic film.

### RELATED ART DOCUMENTS

### PATENT DOCUMENTS

Patent document 1: Japanese Patent No. 5980355
Patent document 2: Japanese Patent No. 5967736

### SUMMARY OF THE INVENTION

### PROBLEMS TO BE SOLVED BY THE INVENTION

It is an object of the present invention to provide an absorbent article using non-stretchable sheets and an elastic film, which reduces the extension stress of the absorbent article to make it easy to stretch, improves the air permeability, increases the maximum elongation of the absorbent article, and prevents a large wrinkle continuous in the direction perpendicular to the stretchable direction from occurring and causing stiffness, so as not to impair the softness of the absorbent article.

### MEANS OF SOLVING THE PROBLEMS

To achieve the above object, according to the present invention of claim 1, an absorbent article includes a stretchable region, wherein the stretchable region of the absorbent article is made of an elastic sheet in which a first non-stretchable sheet, an elastic film and a second non-stretchable sheet are laminated in this order, the elastic sheet is provided with a plurality of joining-opening sections that are constituted by joints to join the first non-stretchable sheet and the second non-stretchable sheet and openings to be generated in the elastic film by forming the joints, the joints are formed by welding the first non-stretchable sheet and the second non-stretchable sheet in a state where the elastic film is stretched in a stretchable direction of the stretchable region, a length of the joint in the stretchable direction is shorter than a length of the joint in a direction perpendicular to the stretchable direction, the length of the joint in the stretchable direction is 0.1 to 1.0 mm, the length of the joint in the direction perpendicular to the stretchable direction is 0.3 to 3.0 mm, and some or all of the j oining-opening sections form adjacent joining-opening sections constituted by two or three of the joining-opening sections which are close to each other, the distance between the joints of the adjacent joining-opening sections in the stretchable direction is 0.3 to 1.5 mm, the openings of the adjacent j oining-opening sections are ununited each other, the joining-opening sections in the adjacent joining-opening section are arranged adjacently in a direction inclined with respect to the stretchable direction, and the adjacent joining-opening sections are arranged such that dividing lines to divide the adjacent joining-opening section into two in a direction perpendicular to the direction inclined with respect to the stretchable direction are not aligned on a straight line.

To achieve the above object, according to the absorbent article provided by the present invention of claim 2, the adjacent joining-opening section and a single joining-opening section not forming the adj acent joining-opening section is alternately arranged.

To achieve the above object, according to the absorbent article provided by the present invention of claim 3, the joining-opening sections constituting the adjacent joining-opening section are arranged in a first direction which is inclined with respect to the stretchable direction, the other joining-opening sections constituting the other adjacent joining-opening section are also arranged in a second direction which is inclined with respect to the stretchable direction and is different from the first direction, the adjacent joining-opening sections are arranged such that dividing lines to divide the adjacent joining-opening section into two in a direction perpendicular to the first direction are not aligned on a straight line, and the other adjacent joining-opening sections are arranged such that dividing lines to divide the other adjacent joining-opening section into two in a direction perpendicular to the second direction are not aligned on a straight line.

To achieve the above object, according to the absorbent article provided by the present invention of claim 4, the second direction is a direction in which the first direction is inverted with a direction perpendicular to the stretchable direction as an axis.

To achieve the above object, according to the absorbent article provided by the present invention of claim 5, the adjacent joining-opening sections constituted by the joining-opening sections arranged in the first direction and the other adjacent joining-opening sections constituted by the other joining-opening sections arranged in the second direction are evenly arranged.

### EFFECTS OF THE INVENTION

According to the absorbent article of the present invention of claim 1, it is possible to reduce the extension stress of the elastic sheet of the absorbent article to make it easy to stretch, improve the air permeability, and increase the maximum elongation of the absorbent article.

According to the absorbent article of the present invention of claim 2, it is further possible to increase the maximum elongation of the elastic sheet, strengthen the tensile strength of the elastic film, or at least prevent the tensile strength of the elastic film from decreasing.

According to the absorbent article of the present invention of claim 3, it is further possible to increase the maximum elongation of the elastic sheet and strengthen the tensile strength in the direction perpendicular to the stretchable direction of the elastic film.

According to the absorbent article of the present invention of claim 3, it is further possible to prevent the tensile strength of the elastic film from decreasing in the direction perpendicular to the first direction.

According to the absorbent article of the present invention of claim 7, it is further possible to make the tensile strength of the elastic film more uniform.

According to the absorbent article of the present invention of claim 1, it is further possible to prevent the tensile strength of the elastic film from decreasing in the direction perpendicular to the second direction.

According to the absorbent article of the present invention of claim 9, it is further possible to make the tensile strength of the elastic film more uniform.

### BRIEF DESCRIPTION OF THE DRAWINGS

FIG. 1 is a perspective view of an underpants-type absorbent article of the present invention in a worn state;
FIG. 2 is a front view of the underpants-type absorbent article of FIG. 1 in a state where the ventral member and the dorsal member are layered and the elastic sheet is extended with the ventral member facing forward;
FIG. 3 is a front view of the underpants-type absorbent article of FIG. 1 with the side sealed section joining the ventral member and the dorsal member peeled apart and developed with the ventral member facing forward;
FIG. 4 is an enlarged cross-sectional view taken along line Y-Y of FIG. 3;
FIG. 5 is an enlarged cross-sectional view taken along line X1-X1 of FIG. 3;
FIG. 6A and 6B are enlarged cross-sectional views taken along line X2-X2 of FIG. 3;
FIG. 7 is a partially enlarged front view of the ventral panel 20 (elastic sheet) in a non-stretched state;
FIG. 8A, 8B and 8C are partially enlarged views of the joining-opening sections shown in Fig.7;
FIG. 9 is an enlarged cross-sectional view taken along line X3-X3 of FIG. 7;
FIG. 10A is a cross-sectional view taken along line A1-A1 of FIG. 8A; FIG. 10B is a cross-sectional view taken along line A2-A2 of FIG. 8B; and FIG. 10C is a cross-sectional view taken along line A3-A3 of FIG. 8C;
FIG.11 is a partially enlarged front view of the ventral panel 20 (elastic sheet) in a non-stretched state provided with joining-opening sections and adjacent joining-opening sections of patterns different from those of the joining-opening sections and the adjacent joining-opening sections shown in FIG.7;
FIG. 12A, 12B and 12C are partially enlarged views of the joining-opening sections shown in FIG.11;
FIG. 13 is a cross-sectional view taken along line A4-A4 of FIG. 12C;
FIG. 14 is a partially enlarged front view of a sample A in a non-stretched state;
FIG. 15 is a partially enlarged front view of a sample B in a non-stretched state;
FIG. 16 is a partially enlarged front view of a sample C in a non-stretched state;
FIG. 17 is a partially enlarged front view of a sample D in a non-stretched state; FIG. 18 is a graph showing a relationship between strain and test force (stress) during continuous tension for a test piece of sample A and a test piece of sample C;
FIG. 19 is a graph showing a relationship between strain and test force (stress) during continuous restoration (contraction) for a test piece of sample A and a test piece of sample C;
FIG. 20 is a graph showing a relationship between strain and test force (stress) during continuous tension to near the elastic limit for a test piece of sample B and a test piece of sample D;
FIG. 21 is a graph showing a relationship between strain and test force (stress) during continuous restoration (contraction) from the tensile state near the elastic limit for a test piece of sample B and a test piece of sample D; and
FIG. 22A is an enlarged front view showing a part of the stretchable region of the outer member when the absorbent article of Patent Document 1 is attached, and FIG. 22B is an enlarged front view showing a part of the stretchable region of the outer member when the absorbent article of Patent Document 2 is attached.

### MODE FOR CARRYING OUT THE INVENTION

### [Basic constitution of underpants-type absorbent article]

An embodiment of the present invention applied to underpants-type absorbent article will be described. First, basic constitution of the underpants-type absorbent article is explained.

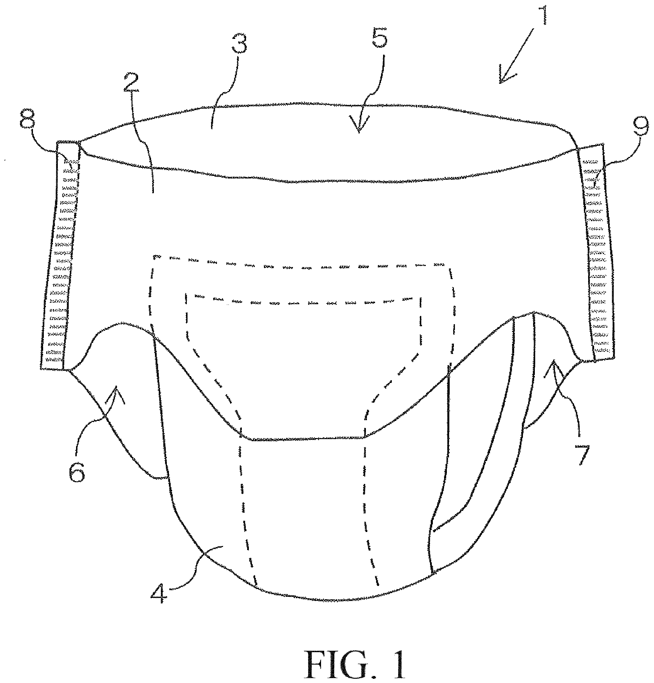

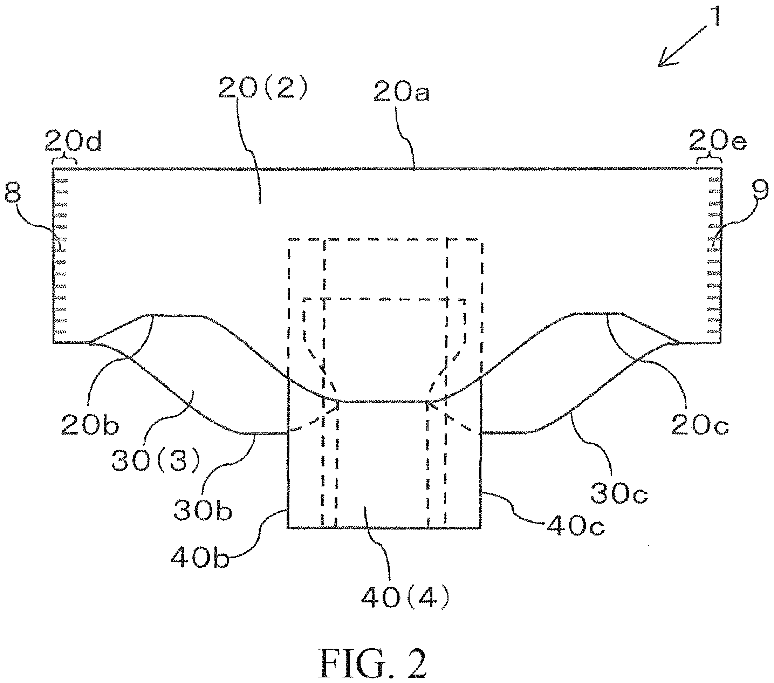

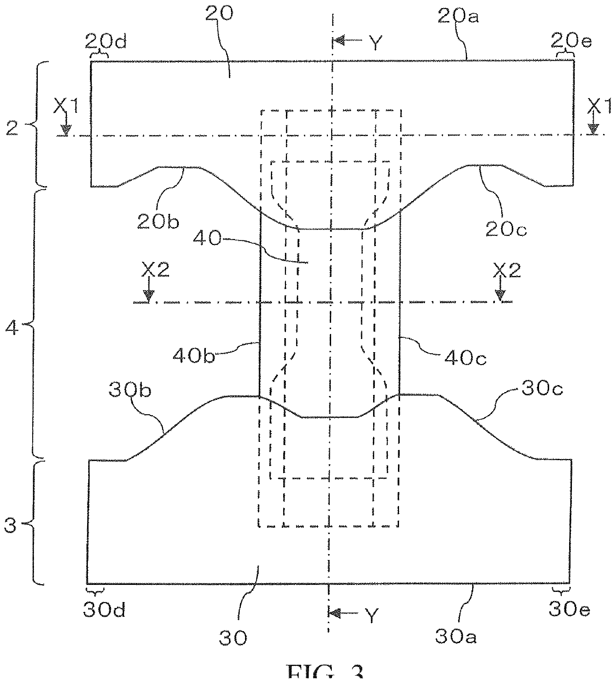

FIG. 1 is a perspective view of an underpants-type absorbent article of the present invention in a worn state; FIG. 2 is a front view of the underpants-type absorbent article of FIG. 1 in a state where the ventral member and the dorsal member are layered and the elastic sheet is extended with the ventral member facing forward; and FIG. 3 is a front view of the underpants-type absorbent article of FIG. 1 with the side sealed section joining the ventral member and the dorsal member peeled apart and developed with the ventral member facing forward.

In the figure, 1 is an underpants-type absorbent article, 2 is a ventral member, 3 is a dorsal member, 4 is a crotch member, 5 is a waist opening, 6 and 7 are leg openings, 8 and 9 are side sealed sections, 20 is a ventral panel, 20a is an upper edge, 20b and 20c is lower edges, 20d is a left end section, 20e is a right end section, 30 is a dorsal panel, 30a is an upper edge, 30b and 30c are lower edges, 30d is a left end section, 30e is a right end section, 40 is a pad, and 40b and 40c are side edges.

As shown in FIG. 1 to FIG. 3, the underpants-type absorbent article 1 is constituted by the ventral member 2, the dorsal member 3, and the crotch member 4. The left end section 20d and the right end section 20e of the ventral panel 20 constituting the ventral member 2 are partially joined by heat sealing, ultrasonic sealing or other heat welding means to form the side sealed section 8. The left end section 30d and the right end section 30e of the dorsal panel 30 constituting the dorsal member 3 are partially joined by heat sealing, ultrasonic sealing or other heat welding means to form the side sealed section 9.

Further, an upper part and an lower part of the pad 40 are respectively adhered to a central portion in a left-right direction of the ventral panel 20 and the dorsal panel 30 with an adhesive or the like, and then the crotch member 4 is formed.

The waist opening 5 is formed by the upper edge 20a of the ventral panel 20 and the upper edge 30a of the dorsal panel 30. The leg opening 6 is formed by the lower edge 20b of the ventral panel 20, the lower edge 30b of the dorsal panel 30 and the side edge 40b of the pad 40. The leg opening 7 is formed by the lower edge 20c of the ventral panel 20, the lower edge 30c of the dorsal panel 30 and the side edge 40c of the pad 40.

The ventral panel 20 has a shape in which the lower edge 20b and the lower edge 20c are dented upward and the central portion is convex downward. The dorsal panel 30 has a shape in which the lower edge 30b and the lower edge 30c are convex downward and the central portion is dented upward. The ventral panel 20 and the dorsal panel 30 are not limited to these shapes, and they can take various shapes such as a rectangular shape and a substantially circular arc shape. The ventral panel 20 and the dorsal panel 30 are not limited to these shapes and may have various shapes such as a rectangular shape and a substantially circular arc shape.

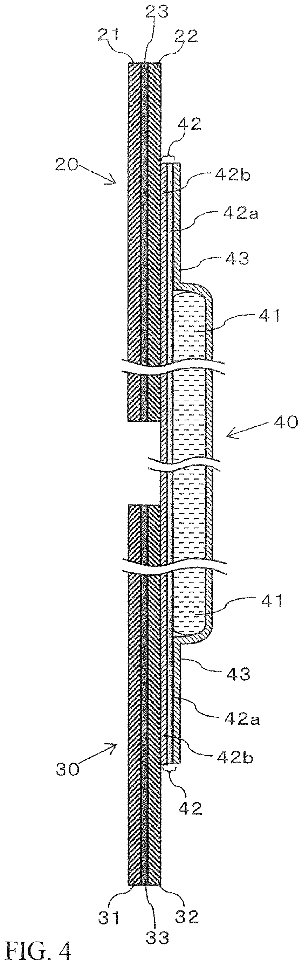

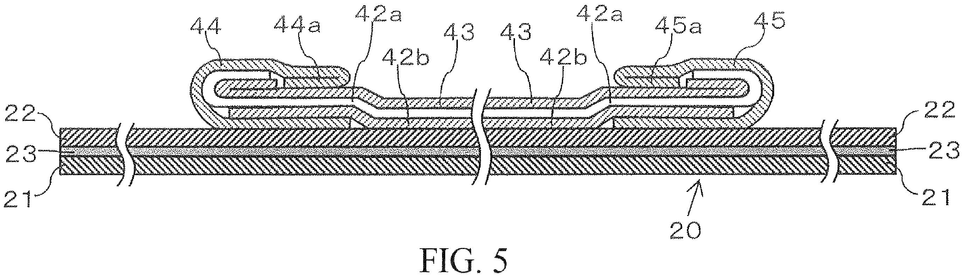

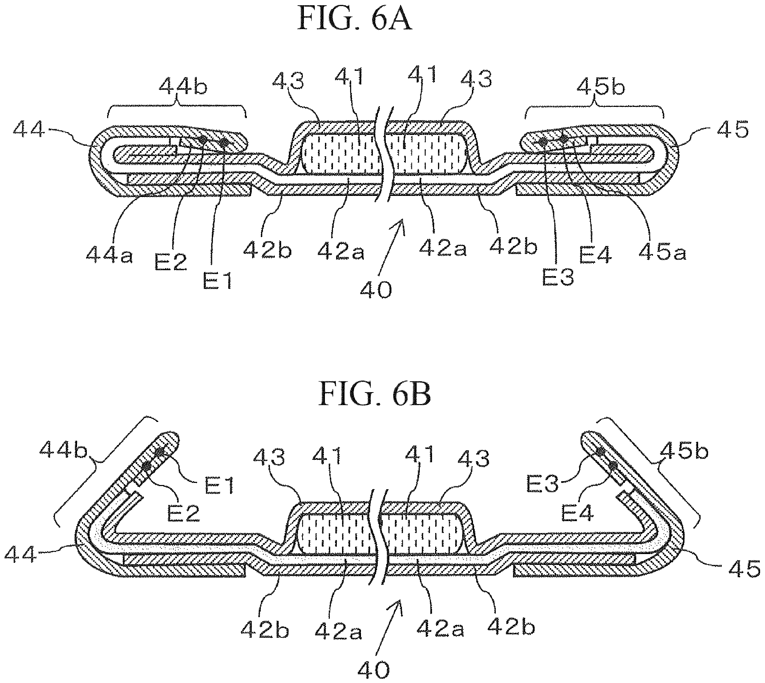

FIG. 4 is an enlarged cross-sectional view taken along line Y-Y of FIG. 3; FIG. 5 is an enlarged cross-sectional view taken along line X1-X1 of FIG. 3; and FIG. 6 is an enlarged cross-sectional view taken along line X2-X2 of FIG. 3.

In the figure, 21 and 31 are first non-stretchable sheets. 22 and 32 are second non-stretchable sheets. 23 and 33 are elastic films. 41 is an absorber. 42 is an outer sheet. 42a is a polyethylene film. 42b is a nonwoven fabric. 43 is an inner sheet. 44 and 45 are gather sheets. 44a and 45a are end sections. 44b and 45b are standing sections. E1, E2, E3, and E4 are elastic members.

As shown in FIG. 4, the ventral panel 20 is constituted by an elastic sheet in which the first non-stretchable sheet 21, the elastic film 23 and the second non-stretchable sheet 22 are laminated in this order. Similarly, the dorsal panel 30 is constituted by an elastic sheet in which the first non-stretchable sheet 31, the elastic film 33 and the second non-stretchable sheet 32 are laminated in this order.

As shown in FIGS. 4 to 6, the pad 40 includes the absorber 41, the outer sheet 42 covering the absorber 41 from the outside (non-skin facing surface), the inner sheet 43 covering the absorber 41 from the inside (skin facing surface), and the gather sheets 44 and 45 respectively attached to the left side portion and the right side portion of the outer sheet 42.

The absorber 41 is mainly composed of superabsorbent polymer particles or mainly composed of cotton-like pulp containing superabsorbent polymer particles, and it is combining absorbent paper (not shown) and the like, and it absorbs and holds body fluid and the like discharged by a user. As shown by broken lines in FIGS. 1 to 3, the absorber 41 has a shape in which the upper portion and the lower portion bulge in the left-right direction, but it can have various shapes such as a rectangular shape.

The outer sheet 42 is formed by laminating the liquid-impermeable polyethylene film 42a and the nonwoven fabric 42b. The nonwoven fabric 42b is laminated to improve the appearance and texture, and the outer sheet 42 may be constituted by only the polyethylene film 42a. The inner sheet 43 is made of a nonwoven fabric.

The outer sheet 42 and the inner sheet 43 overlap outside the periphery of the absorber 41 and are bonded to each other by an adhesive (not shown) or thermal fusion at the overlapping portion, whereby the absorber 41 is enclosed.

As shown in FIG. 5 and FIG. 6, in the pad 40, the left end section 44a of the gather sheet 44 is folded inward and adhered to the overlapped surface of the gather sheet 44, and the right end section 45a of the gather sheet 45 is folded inward and adhered to the overlapped surface of the gather sheets 45. A left inside section of the gather sheet 44 (which is inside the folded and overlapped section by the left end section 44a) is bonded by adhesive (not shown) or thermal fusion to the left side section of the outer sheet 42 opposite to the inner sheet 43, and a section inside the folded and overlapped section of the right end section 45a of the gather sheet 45 is bonded to the right side section of the outer sheet 42 opposite to the inner sheet 43 by an adhesive (not shown) or thermal fusion bonding. Left end sections of the outer sheet 42 (polyethylene film 42a) and the inner sheet 43 are folded inward with the left end section of the gather sheet 44, and right end sections of the outer sheet 42 (polyethylene film 42a) and the inner sheet 43 are folded inward with the right end section of the gather sheet 45.

As shown in FIG. 5, the upper and lower portions of the outer surfaces (the surfaces not bonded to the gather sheets 44 and 45) of the end sections 44a and 45a of the gather sheets 44 and 45 are adhered to the inner sheet 43. As shown in FIG. 6A, elastic members E1, E2, E3, and E4 such as elastic strands are provided at portions which are folded inside the end sections 44a and 45a of the gather sheets 44 and 45 and are not adhered to the inner sheet 43, and then standing sections 44b and 45b are formed.

The elastic members E1 to E4 are bonded and fixed in an extended state in the vertical direction (longitudinal direction). As shown in FIG. 6B, as the elastic members E1 to E4 shrink, the standing sections 44b and 45b of the gather sheets 44 and 45 stand at an acute angle to form a standing gather. By forming the standing gathers in this manner, leakage of body fluid is prevented.

The configurations of the outer sheet 42, the inner sheet 43, and the gather sheets 44 and 45 are not limited to the above, and other configurations can be adopted.

### [Elastic sheet]

Referring to FIG. 4, the elastic sheet constitutes the ventral panel 20 and the dorsal panel 30. The elastic sheets are formed by laminating the first non-stretchable sheets 21 and 31, the elastic films 23 and 33, and the second non-stretchable sheets 22 and 32 in this order.

The first non-stretchable sheets 21, 31 and the second non-stretchable sheets 22, 32 can use breathable and soft non-stretchable sheets, for example nonwoven fabric.

As the raw fiber of this nonwoven fabric, for example, polyolefin fiber such as polyethylene fiber and polypropylene fiber, polyester fiber, polyamide fiber, rayon fiber, natural fiber such as cotton, etc., or mixed fibers or composite fibers using two or more of these can be used. As a method for producing the nonwoven fabric, a known method such as spunbond method, melt blow method, thermal bond method, needle punch method, spunlace method, or the like can be used, and furthermore, a combination of these manufacturing methods may be used.

In this case, the first non-stretchable sheets 21, 31 and the second non-stretchable sheets 22, 32 may use the same nonwoven fabric, different nonwoven fabrics, and a laminate of two or more nonwoven fabrics.

The first non-stretchable sheets 21, 31 and the second non-stretchable sheets 22, 32 have some stretchability, for example, stretchability of not more than 150% (doesn't break at 150% stretch) or less.

In particular, a spunlace nonwoven fabric mixed with sweat-absorbant fiber such as cotton and rayon and thermoplastic fiber such as polypropylene, spunbond nonwoven fabric using soft material such as polyethylene, and the like have lower tensile strength than nonwoven fabric mainly made of general polypropylene or the like and are easy to stretch and extend by 103 to 150%. However, when such material is manufactured by conventional method in which elastic members such as elastic strands are arranged in the left-right direction (waistline direction), it cannot be used because the strength of the product is insufficient. By laminating the above material with the elastic film, it has strength to a certain degree and can be suitably used as the material which adds sweat absorptive and softness to the elastic sheet.

As the elastic films 23 and 33, it is possible to use a resin film having elasticity, for example, one obtained by processing one kind or two or more kinds of thermoplastic compositions such as polystyrene elastomer, polyolefin elastomer, polyester elastomer, polyamide elastomer, silicone elastomer, polyvinyl chloride elastomer, polyurethane elastomer, and the like into a film shape or a laminate thereof.

In addition, it is preferable that the elastic films 23 and 33 have a maximum elongation 200% or more.

### [Joint and opening in elastic sheet]

In the elastic sheet constituting the ventral panel 20 and the dorsal panel 30, the first non-stretchable sheets 21, 31 and the second non-stretchable sheets 22, 32 are joined by a large number of joints.

The multiple joints are formed by applying a predetermined pattern of heat sealing and ultrasonic sealing to the first non-stretchable sheets 21, 31 and the second non-stretchable sheets 22, 32 sandwiching the elastic films 23, 33 elongated in the left-right direction. That is, the sealing surfaces of the first non-stretchable sheets 21, 31 and the second non-stretchable sheets 22, 32 are thermally welded to form a large number of joints.

The joint preferably has a long vertically elongated shape in the vertical direction (direction perpendicular to the stretchable direction), for example, a rectangular shape, an oval shape, a drum shape, and the like. The lateral length (width) of the joint is preferably 0.1 to 1.0 mm, particularly preferably 0.1 to 0.5 mm. The vertical length of the joint is preferably 0.3 to 3.0 mm, particularly preferably 0.5 to 1.5 mm. If the length in the left-right direction is less than 0.1 mm or the length in the vertical direction is less than 0.3 mm, the bonding strength between the first non-stretchable sheet and the second non-stretchable sheet becomes extremely low. If the length in the left-right direction is larger than 1.0 mm or the length in the vertical direction is larger than 3.0 mm, the tensile strength of the elastic sheet is extremely lowered.

In this case, the vertically elongated shape long in the vertical direction (the direction perpendicular to the stretchable direction) is not strictly limited to the vertically elongated shape in the vertical direction. Even if it is slightly inclined from the vertical direction, for example, even if it is inclined about 20 degrees with respect to the vertical direction, it may be any shape so long as it is vertically elongated. Also, the vertically elongated shape may be not only a linear shape but also a curved shape such as a longitudinally long waveform.

Since the extension stress, maximum elongation, and tensile strength of the elastic films 23 and 33 are different depending on the material and thickness of the elastic films 23 and 33, the shape and dimensions of the joint are set so that the elastic sheet having desirable extension stress, maximum elongation, tensile strength and texture can be obtained according to the material, thickness and the like of the elastic films 23 and 33.

When this joint is formed, the elastic films 23 and 33 are melted at the portion subjected to heat sealing and ultrasonic sealing, the circumference thereof is broken, and opening is formed.

As a result, many joining-opening sections consisting of joint and opening are formed in the elastic sheet (ventral panel 20, dorsal panel 30).

After forming the joints of the first non-stretchable sheets 21, 31 and the second non-stretchable sheets 22, 32 in the elastic sheet (ventral panel 20, dorsal panel 30), when the elongated state of the elastic films 23, 33 is released, the elastic sheet shrinks due to the contractile force of the elastic films 23, 33 and then elasticity is given to the elastic sheet.

In this case, since the pad 40 has little elasticity, the elasticity and the maximum elongation of the portion where the pad 40 is attached to the ventral panel 20 and the dorsal panel 30 are reduced. As the side sealed sections 8 and 9 also have large sealed area, their elasticity and maximum elongation are reduced.

Therefore, the ventral panel 20 and the dorsal panel 30 become stretchable regions functioning as elastic sheets, except for the portion to which the pad 40 is attached and the left end section 20d, 30d and the right end section 20e, 30e which become the side sealed sections 8 and 9.

### [Example of joining-opening section and adjacent joining-opening section]

Next, an example of a joining-opening section and an adjacent joining-opening section will be described.

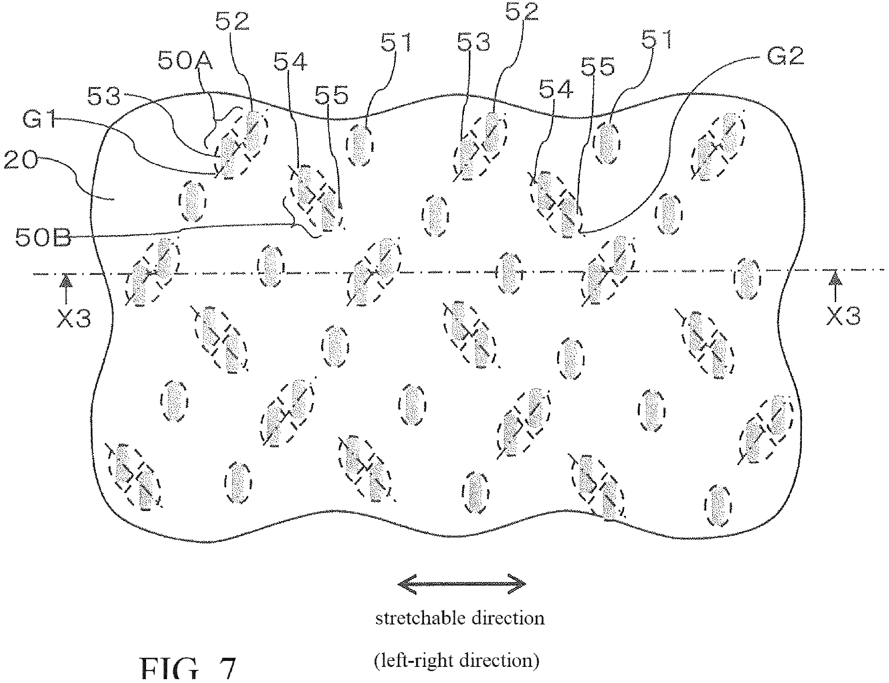

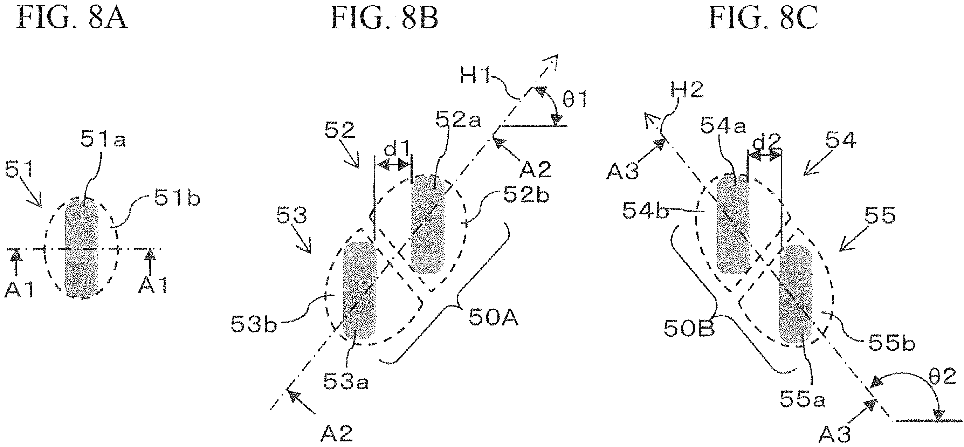

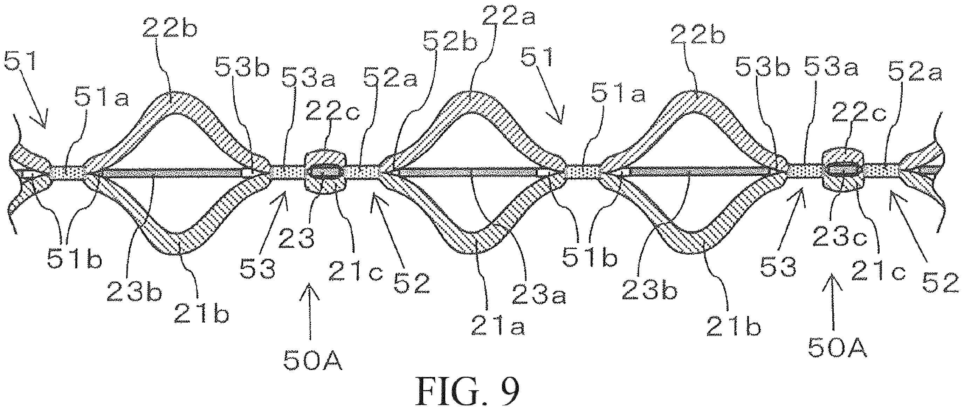

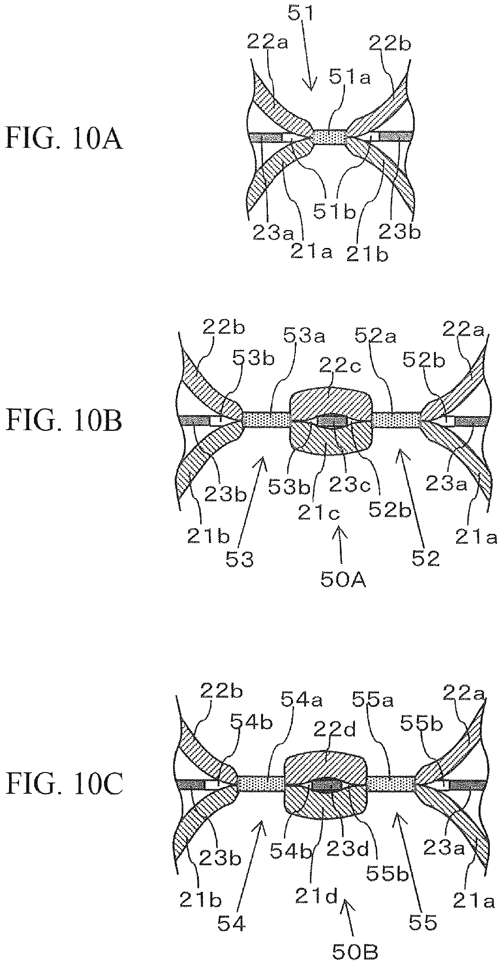

FIG. 7 is a partially enlarged front view of the ventral panel 20 (elastic sheet) in a non-stretched state; FIG. 8 is an enlarged view of the joining-opening sections shown in Fig.7; FIG. 9 is an enlarged cross-sectional view taken along line X3-X3 of FIG. 7; FIG. 10A is a cross-sectional view taken along line A1-A1 of FIG. 8A; FIG. 10B is a cross-sectional view taken along line A2-A2 of FIG. 8B; and FIG. 10C is a cross-sectional view taken along line A3-A3 of FIG. 8C.

In the figures, 51 to 55 are joining-opening sections, 51a to 55a are joints, 51b to 55b are openings, 50Aand 50B are adjacent joining-opening sections, G1 and G2 are division lines, H1 is a first direction, and H2 is a second direction.

As shown in FIGS. 7, 8 and 10, the ventral panel 20 (elastic sheet) has five types of joining-opening sections which are a joining-opening section 51 constituted by a vertically elongated joint 51a and an elliptical opening 51b (in FIG. 8A and FIG. 10A), a joining-opening section 52 constituted by a vertically elongated joint 52a and opening 52b, a joining-opening section 53 constituted by a vertically long joint 53a and an opening 53b (in FIG. 8B and FIG. 10 B), a joining-opening section 54 constituted by a vertically elongated joint 54a and an opening 54b, and a joining-opening section 55 constituted by a joint 55a and an opening 55b in a vertically long shape (in FIG. 8C and FIG. 10C).

The joining-opening section 52 and the joining-opening section 53 are close to each other and constitute an adjacent joining-opening section 50A. The joining-opening section 54 and the joining-opening section 55 are close to each other and constitute an adjacent joining-opening section 50B.

The distance d1 between the joints 52a and 53a in the left-right direction in the adjacent joining-opening section 50A is preferably 0.2 to 2.5 mm, especially 0.3 to 1.5 mm. The same is true of the distance d2 between the joints 54a and 55a in the left-right direction in the adjacent joining-opening section 50B. When the intervals d1, d2 are less than 0.2 mm, the opening 52b (54b) and 53b (55b) are united and the adjacent joining-opening section 50A (50B) is not formed. When the intervals d1, d2 are larger than 2.5 mm, the effect of the adjacent joining-opening section described later will not be exerted.

In the adjacent joining-opening section 50A, the joining-opening section 52 and the joining-opening section 53 are arranged in the first direction H1 inclined to the right upwardly with respect to the stretchable direction (left-right direction) of the elastic sheet, and the opening 52b and 53b are shaped such that the ellipse whose vertical axis is inclined in the first direction H1 is divided into two. In this case, an inclination angle θ1 in the first direction H1 is preferably 15 to 75 degrees, particularly preferably 20 to 60 degrees.

In the adjoining joining-opening section 50B, the joining-opening section 54 and the joining-opening section 55 are arranged in the second direction H2 inclined leftward and upward with respect to the stretchable direction (left-right direction) of the elastic sheet, and the opening 54b and 54b are shaped such that the ellipse whose vertical axis is inclined in the second direction H2 is divided into two. The second direction H2 is a direction in which the first direction H1 is inverted with the direction perpendicular to the stretchable direction (left-right direction) of the elastic sheet as an axis, and an inclination angle Θ2 in the second direction H2 is 105 to 165 degrees, and particularly preferably 120 to 160 degrees.

As shown in FIG. 7, in the ventral panel 20 (elastic sheet), a left-right direction row in which the adjacent joining-opening section 50A and the joining-opening section 51 are alternately arranged is formed, a left-right direction row in which the adjacent joining-opening section 50B and the joining-opening section 51 are alternately arranged is formed, and these rows are alternately arranged in a direction perpendicular to the left-right direction.

In this case, the adjacent joining-opening sections 50A are arranged so that dividing lines G1 for dividing the adjacent joining-opening section 50A in the direction perpendicular to the first direction H1 are not aligned on a straight line, and the adjacent joining-opening sections 50B are arranged so that the dividing lines G2 dividing the adjacent joining-opening section 50 B in the direction perpendicular to the second direction H2 are not aligned on a straight line.

Similar to the ventral panel 20, the dorsal panel 30 has joining-opening sections 51 to 55 include joints 51a to 55a and openings 51b to 55b, and the joining-opening section 50A is formed from joining-opening sections 52 and 53, and the joining-opening section 50B is formed from joining-opening sections 54 and 55.

As shown in FIGS. 10A to 10C, in the joints 51a to 55a, the first non-stretchable sheet 21, the second non-stretchable sheet 22 and the elastic film 23 are melted and solidified, and the first non-stretchable sheet 21 and the second non-stretchable sheet 22 are welded.

Here, the first non-stretchable sheet 21, the second non-stretchable sheet 22, and the elastic film 23 positioned on the left side of the joint 51a are respectively set as the first non-stretchable sheet 21a, the second non-stretchable sheet 22a, and the elastic film 23a. The first non-stretchable sheet 21, the second non-stretchable sheet 22, and the elastic film 23 positioned on the right side of the joint 51a are respectively set as the first non-stretchable sheet 21b, the second non-stretchable sheet 22b, and the elastic film 23b (referring to FIG. 10A). The first non-stretchable sheet 21, the second non-stretchable sheet 22, and the elastic film 23 positioned between the joints 52a and 53a are respectively set as the first non-stretchable sheet 21c, the second non-stretchable sheet 22c, and the elastic film 23c (referring to FIG. 10B). The first non-stretchable sheet 21, the second non-stretchable sheet 22, and the elastic film 23 positioned between the joints 54a and 55a are respectively set as first non-stretchable sheet 21d, second non-stretchable sheet 22d, and elastic film 23d (referring to FIG. 10C).

As shown in FIG. 9, in the left-right direction row in which the adjacent joining-opening section 50A and the joining-opening section 51 are alternately arranged, after the joints 51a to 53a are formed, the contraction of the elastic film 23a, 23b in the left-right direction causes the first non-stretchable sheet 21a, 21b and the second non-stretchable sheet 22a, 22b protrude in directions away from each other, and wrinkles extending in the vertical direction are formed in a portion between the adjacent joining-opening section 50A and the joining-opening section 51. In the left-right direction row where the adjacent joining-opening section 50B and the joining-opening section 51 are alternately arranged, similar wrinkles are formed in a portion between the adjacent joining-opening section 50B and the joining-opening section 51.

In this case, as shown in FIG. 7, the ventral panel 20 (elastic sheet) has a portion between the adjacent joining-opening section 50A and the joining-opening section 51 and a portion between the adjacent joining-opening section 50B and the joining-opening section 51, which are not continuous in the vertical direction. Wrinkles formed in this part also do not continue in the vertical direction. Therefore, stiffness dose not occur due to large wrinkles continuing in the vertical direction, and softness of the ventral panel 20 (elastic sheet) is prevented from being impaired.

On the other hand, since the joining-opening section 52 and the joining-opening section 53 are close to each other in the adjacent joining-opening section 50A, the first non-stretchable sheet 21c and the second non-stretchable sheet 22c located between the joints 52a and 53a overlap with the elastic film 23c located between the openings 52b and 53b. Similarly, also in the adjacent joining-opening section 50B, the first non-stretchable sheet 21d and the second non-stretchable sheet 22d located between the joints 54a and 55a overlap with the elastic film 23d located between the opening portions 54b and 55b.

Therefore, when the ventral panel 20 (elastic sheet) is pulled in the left-right direction, the elastic film 23 (23a , 23b) located between the adjacent joining-opening sections 50A, 50B and the joining-opening section 51 is extended, wrinkles formed by the non-stretchable sheet 21 (21a, 21b) and the second non-stretchable sheet 22 (22a, 22b) is extended, and the ventral panel 20 is extended.

The dorsal panel 30 is extended in the left-right direction in the same way as the ventral panel 20.

The elastic sheet provided with only the joining-opening sections 51 without the adjacent joining-opening sections 50A and 50B may narrow the interval of the joining-opening sections 51 or enlarge the joining-opening section 51 to reduce the extension stress of the elastic sheet to make it easier to stretch and improve the air permeability, but it cannot make the maximum elongation of the elastic sheet greater than a certain value, so its function as an absorbent article is inadequate.

On the other hand, the elastic sheet provided with the adjacent joining-opening sections 50A and 50B can narrow the interval between the joining-opening section 51 and the adjacent joining-opening section 50A (50B) or the interval between the adjacent joining-opening sections 50A and 50B or enlarge the adjacent joining-opening section 50A (50B) or the joining-opening sections 52 to 55, so that the adjacent joining-opening section 50A (50B) widens as well as one large joining-opening section joining the joining-opening sections 52 and 53 (54 and 55) to reduce the extension stress of the elastic sheet to make it easier to stretch and improve the air permeability. Furthermore, the size of the joining-opening section 52, 53 (54, 55) of the adjacent joining-opening section 50A (50B) is about half of the size of one large joining-opening section which joined the joining-opening section 52, 53 (54, 55). Therefore, it is difficult to plastic deformation even if the elastic film is greatly extended, compared with the elastic sheet with the large joining-opening section, so that the maximum elongation of the elastic sheet can be made larger than a certain value.

As shown in FIG. 7, the adjacent joining-opening section 50Aor 50B and the separate joining-opening section 51 are arranged alternately in the left-right direction, so that it is possible to increase the maximum elongation of the elastic sheet and prevent from decreasing the tensile strength of the elastic film.

In addition, when the joining-opening sections 52 and 53 and joining-opening sections 54 and 55 are arranged to be inclined with respect to the stretchable direction like the adjacent joining-opening sections 50A and 50B, as compared with the arrangement without inclination, the tensile strength (tear strength) of the elastic film in the direction perpendicular to the stretchable direction which tends to be pulled when wearing the article becomes strong.

Furthermore, when the joining-opening section of the adjacent joining-opening section is arranged to be inclined in one direction, the tensile strength (tear strength) of the elastic film in the direction perpendicular to the inclined direction decreases, but the second direction H2 in which the joining-opening sections 54, 55 are inclined is inverted to the first direction H1 in which the joining-opening sections 52, 53 are inclined, and the adjacent joining-opening sections 50A and 50B are arranged evenly, so that it is possible to prevent the tensile strength (tear strength) of the elastic film in the direction perpendicular to the inclined direction (the first direction H1 and the second direction H2) from decreasing and keep the tensile strength of the whole elastic film constant.

In addition, when the dividing line G1 of the adjacent joining-opening section 50A is not aligned on a straight line, it is possible to prevent the tensile strength (tear strength) of the elastic film in the direction perpendicular to the first direction H1 from decreasing. Further, when the dividing line G2 of the adjacent joining-opening section 50B is not aligned on a straight line, it is possible to prevent the tensile strength (tear strength) of the elastic film in the direction perpendicular to the second direction H2 from decreasing.

### [Other example of joining-opening section and adjacent joining-opening section]

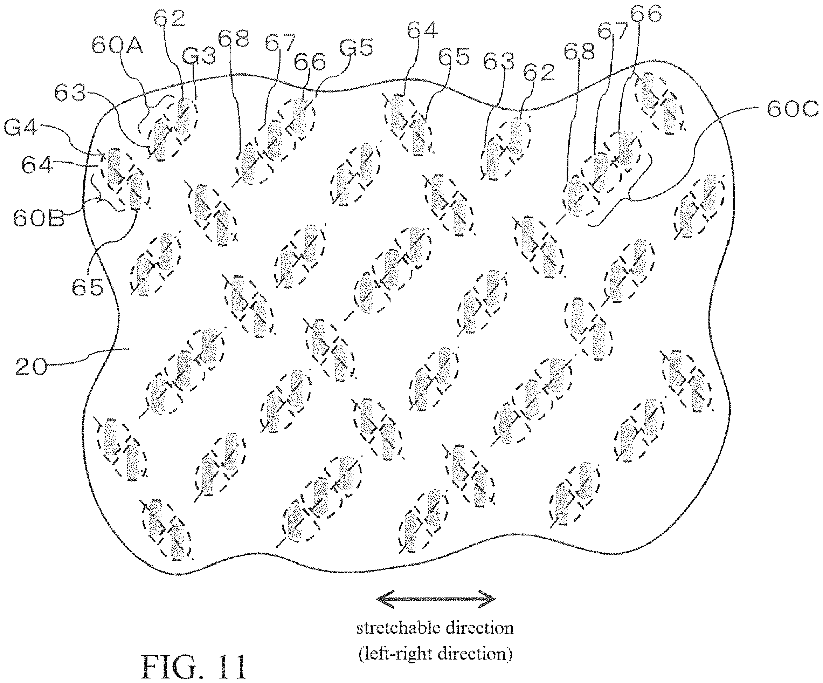

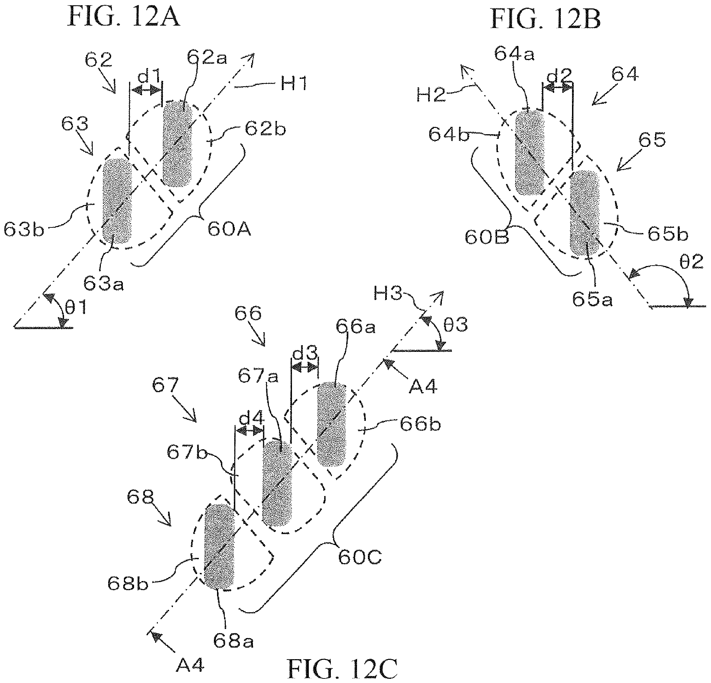

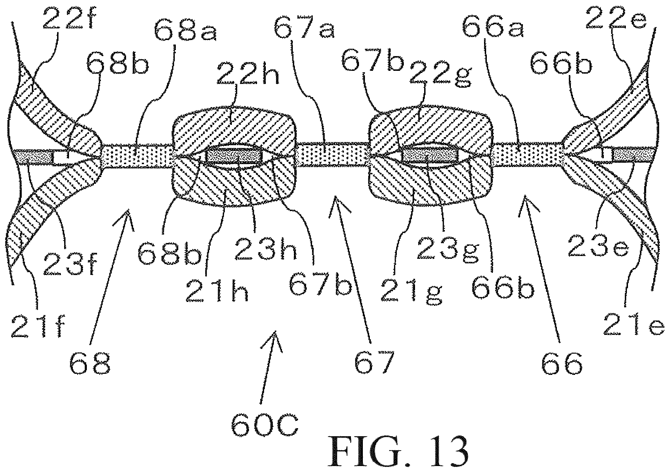

FIG.11 is a partially enlarged front view of the ventral panel 20 (elastic sheet) in a non-stretched state provided with joining-opening sections and adjacent joining-opening sections of patterns different from those of the joining-opening sections and the adjacent joining-opening sections shown in FIG.7; FIG. 12A, 12B and 12C are partially enlarged views of the joining-opening sections shown in FIG.11; and FIG. 13 is a cross-sectional view taken along line A4-A4 of FIG. 12.

In the figures, 62 to 68 are joining-opening sections, 62a to 68a are joints, 62b to 68b are openings, 60A, 60B and 60C are adjacent joining-opening sections, G3 to G5 are division lines, and H3 is a third direction.

As shown in FIGS. 11 and 12, the ventral panel 20 (elastic sheet) has seven types of joining-opening sections which are a joining-opening section 62 constituted by a vertically elongated joint 62a and an opening 62b, a joining-opening section 63 constituted by a vertically elongated joint 63a and an opening 63b (in FIG. 12A), a joining-opening section 64 constituted by a vertically elongated joint 64a and an opening 64b, a joining-opening section 65 constituted by a vertically elongated joint 65a and an opening 65b (in FIG. 12B), a joining-opening section 66 constituted by a vertically elongated joint 66a and an opening 66b, a joining-opening section 67 constituted by a vertically elongated joint 67a and an opening 67b, and a joining-opening section 68 constituted by a vertically elongated joint 68a and an opening 68b (in FIG. 12C).

Of these, the joining-opening sections 62 to 65 are the same as the joining-opening sections 52 to 55 shown in FIGS. 7, 8B and 8C, and the sizes and shapes of the joints 62a to 68a are the same as the sizes and shapes of the joints 51a to 55a shown in FIG. 8.

The joining-opening section 62 and the joining-opening section 63 are close to each other to form the adjacent joining-opening section 60A. The joining-opening section 64 and the joining-opening section 65 are close to each other to form the adjacent joining-opening section 60B. The joining-opening section 66, the joining-opening section 67, and the joining-opening section 68 are close to each other to form the adjacent joining-opening section 60C. The adjacent joining-opening sections 60A and 60B are the same as the adjacent joining-opening sections 50A and 50B.

That is, the joining-opening sections 62 and 63 of the adjacent joining-opening section 60A are arranged in the first direction H1 inclined at an angle θ1 with respect to the stretchable direction (left-right direction) of the elastic sheet, and the interval of them in the left-right direction is d1, and the openings 62b and 63b have such a shape that the ellipse whose vertical axis is inclined in the first direction H1 is divided into two. The joining-opening sections 64 and 65 of the adjacent joining-opening section 60B are arranged in the second direction H2 inclined at an angle Θ2 with respect to the stretchable direction (left-right direction) of the elastic sheet, and the interval of them in the left-right direction is d2, and the openings 64b and 65b have such a shape that the ellipse whose vertical axis is inclined in the first direction H2 is divided into two.

In the adjacent joining-opening section 60C, the distance d3 between the joint 66a and 67a in the left-right direction and the distance d4 between the joint 67a and 68a in the left-right direction are the same, preferably 0.2 to 2.5 mm, particularly 0.3 to 1.5 mm. When the intervals d3 and d4 are less than 0.2 mm, the opening 66b, 67b, 68b are united and the adjacent joining-opening section 60C is not formed. When the intervals d3, d4 are larger than 2.5 mm, the effect of the adjacent joining-opening section will not be exerted.

Also, the joining-opening sections 66, 67, 68 of the adjacent joining-opening section 60C are arranged in the third direction H3 inclined to the right upward with respect to the stretchable direction (left-right direction) of the elastic sheet, and the opening 66b, 67b, 68b have such a shape that the ellipse whose vertical axis is inclined in the third direction H3 is divided into three. In this case, the inclination angle θ3 in the third direction H3 is preferably 15 to 75 degrees, particularly preferably 20 to 60 degrees, and may be the same as or different from the inclination angle θ1 in the first direction H1.

In this case, the adjacent joining-opening sections 60A are arranged such that the dividing lines G3 for dividing the adjacent joining-opening section 60A in the direction perpendicular to the first direction H1 are not aligned on a straight line. The adjacent opening joining-opening sections 60B are arranged so that the dividing lines G4 dividing the adjacent joining-opening section 60B in the direction perpendicular to the second direction H2 are not aligned on a straight line. The adjacent opening joining-opening sections 60C are arranged so that the dividing lines G5 dividing the adjacent joining-opening section 60C in the direction perpendicular to the third direction H3 and the dividing lines G3 are not aligned on a straight line.

As with the ventral panel 20, the dorsal panel 30 is also provided with joining-opening sections 62 to 68 constituted by the joints 62a to 68a and the openings 62b to 68b, and the joining-opening section 60A is formed from the joining-opening sections 62 and 63, and the joining-opening section 60B is formed from joining-opening sections 64 and 65, and the joining-opening section 60C is formed from joining-opening sections 66 and 67 and 68.

Similar to the joints 51a to 55a shown in FIGS. 10A to 10C, in the joints 62a to 68a, the first non-stretchable sheet 21, the second non-stretchable sheet 22 and the elastic film 23 are melted and solidified, and the first non-stretchable sheet 21 and the second non-stretchable sheet 22 are welded.

As shown in FIG. 11, three kinds of adjacent joining-opening sections 60A, 60B and 60C are alternately arranged in the left-right direction in the ventral panel 20. Just as wrinkles are formed in the portion between the adjacent joining-opening section 50A and the joining-opening section 51 (referring to FIG. 9), after the joints 62a to 68a are formed, the elastic film 23 contracts in the left-right direction, so that the first non-stretchable sheet 21 and the second non-stretchable sheet 22 protrude in directions away from each other, and wrinkles extending in the vertical direction are formed in portions located between adjacent joining-opening sections 60A, 60B and 60C.

In this case, as shown in FIG. 11, the portion between each adjacent joining-opening sections 60A, 60B and 60C in the ventral panel 20 (elastic sheet) is not continuous in the vertical direction, so that the wrinkles formed in this portion are also not continuous in the vertical direction, and then the ventral panel 20 (elastic sheet) can be textured finely and flexibly.

Therefore, stiffness dose not occur due to large wrinkles continuing in the vertical direction, and softness of the ventral panel 20 (elastic sheet) is prevented from being impaired.

On the other hand, since the joining-opening section 62 and the joining-opening section 63 in the adjacent joining-opening section 60A are close to each other, both of the first non-stretchable sheet 21 and the second non-stretchable sheet 22 located between joint 62a and 63a overlap with the elastic film 23 located between opening 62b and 63b, as like the adjoining joining-opening section 50 A (see FIG. 10B).

Here, the first non-stretchable sheet 21, the second non-stretchable sheet 22 and the elastic film 23 located on the right side of the joint 66a are respectively set as first non-stretchable sheet 21e, second non-stretchable sheet 22e and elastic film 23e. The first non-stretchable sheet 21, the second non-stretchable sheet 22 and the elastic film 23 located on the left side of the joint 68a are respectively set as the first non-stretchable sheet 21f, the second non-stretchable sheet 22f and the elastic film 23f. The first non-stretchable sheet 21, the second non-stretchable sheet 22 and the elastic film 23 positioned between the joints 66a and 67a are respectively set as the first non-stretchable sheet 21g, the second non-stretchable sheet 22g and the elastic film 23g. The first non-stretchable sheet 21, the second non-stretchable sheet 22 and the elastic film 23 located between the joints 67a and 68a are respectively set as first non-stretchable sheet 21h, second non-stretchable sheet 22h and elastic film 23h.

As shown in FIG. 13, in the adjacent joining-opening section 60C, the first non-stretchable sheet 21e and the second non-stretchable sheet 22e are separated from the elastic film 23e on the right side of the joining-opening section 66, and the first non-stretchable sheet 21f and the second non-stretchable sheet 22f are separated from the elastic film 23f on the left side of the joining-opening section 68. On the other hand, the first non-stretchable sheet 21g and the second non-stretchable sheet 22g located between the joint 66a and 67a overlap with the elastic film 23g located between the opening 66b and the opening 67b, and the first non-stretchable sheet 21h and the second non-stretchable sheet 22h located between joints 67a and 68a overlap with the elastic film 23h located between opening 67b and 68b.

Therefore, when the ventral panel 20 (elastic sheet) is pulled in the left-right direction, the elastic film 23 in the portion located between the adjacent joining-opening sections 60A, 60B and 60C is extended, the wrinkles formed by the first non-stretchable sheet 21 and the second non-stretchable sheet 22 is extend, and the ventral panel 20 is extended.

The dorsal panel 30 is also extended in the left-right direction.in the same way as the ventral panel 20,

When the adjacent joining-opening section 60A, 60B constituted by two joining-opening sections and the adjacent joining-opening section 60C constituted by three joining-opening sections 6 are arranged at a predetermined ratio, the maximum elongation of the elastic sheet can be increased without damaging the tensile strength of the elastic film.

When the dividing lines G3 of the adjacent joining-opening sections 60A and the dividing lines G5 of the adjacent j oining-opening sections 60C are not aligned on a straight line, it is possible to prevent the tensile strength (tear strength) of the elastic film in the direction perpendicular to the first direction H1 from decreasing. When the dividing lines G4 of the adjacent joining-opening section 60B are not aligned on a straight line, it is possible to prevent the tensile strength (tear strength) of the elastic film in the direction perpendicular to the second direction H2 from decreasing.

### [Tensile test of elastic sheet]

Four kinds of elastic sheets of samples A to D were manufactured, and a tensile test (elongation and restoration cycles test) was performed for each sample.

The elastic sheet of each sample is manufactured by sandwiching the elongated elastic film 23 between the first non-stretchable sheet 21 and the second non-stretchable sheet 22, performing ultrasonic sealing with a predetermined pattern to form a large number of joints.

A nonwoven fabric with a weight per unit area of 18 g/m² produced by spunbonding polypropylene as raw material fiber was used as the first non-stretchable sheet 21 and the second non-stretchable sheet 22.

Polyolefin based elastomer and polyurethane based elastomer as a main material processed into a film shape and having a maximum elongation of 300% (natural length is considered to be 100% elongation) was used as the elastic film 23.

In the tensile test, the width of the test piece was 50 mm, the length (distance between chucks when attached to the testing machine) was 100 mm, and the pulling speed was 300 mm/min. After the test piece is elongated to a predetermined length, it was restored (contracted), and the relationship between strain and stress of the test piece was continuously measured during the elongation and restoration (contraction).

"Autograph AGS-X" manufactured by Shimadzu Corporation was used as a tensile testing machine.

### [Sample A]

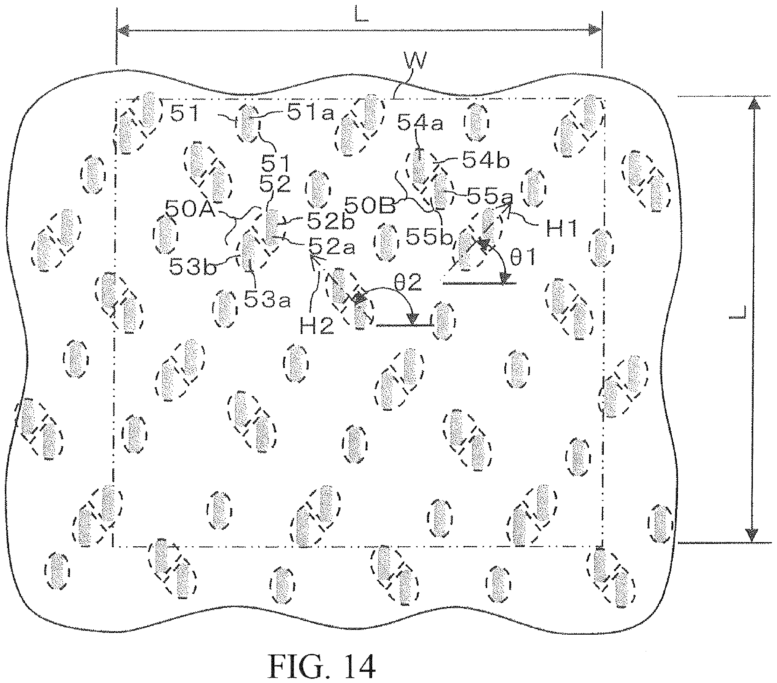

FIG. 14 is a partially enlarged front view of a sample A in a non-stretched state. In the figure, W is a square frame of L (= 10 mm) × L (= 10 mm).

As shown in FIG. 14, the sample A is provided with joints and openings similarly to those of the ventral panel 20 (elastic sheet) shown in FIG. 7.

The sample A is provided with joining-opening section 51 constituted by joint 51a and opening 51b, joining-opening section 52 constituted by joint 52a and opening 52b, joining-opening section 53 constituted by joint 53a and opening 53b, joining-opening section 54 comprising joint 54a and opening 54b, and joining-opening section 55 comprising joint 55a and opening 55b. The joining-opening section 52 and the joining-opening section 53 constitute an adjacent joining-opening section 50A. The joining-opening section 54 and the joining-opening section 55 constitute an adjacent joining-opening section 50B.

The joints 51a to 55a have oval shapes in which the width in the left-right direction is about 0.3 mm and the length in the vertical direction is about 1.0 mm. The distance d1 between the joint 52a and the joint 53a in the adjacent joining-opening section 50A and the distance d2 between the joint 54a and the joint 55a in the adj acent joining-opening section 50B are both about 0.5 mm.

The inclination angle θ1 in the first direction H1 in which the joining-opening section 52 and the joining-opening section 53 are arranged in the adjacent joining-opening section 50A is about 50 degrees. The inclination angle Θ2 in the second direction H2 in which the joining-opening section 54 and the joining-opening section 55 are arranged in the adjacent joining-opening section 50B is about 130 degrees.

The sample A is provided with a row of left-right direction in which joining-opening section 51 and adjacent joining-opening section 50A, 50B are alternately arranged at a rate of 5 per unit length L = 10 mm. Seven such left-right direction rows are provided per unit length L = 10 mm in the vertical direction. 16 pieces of the joining-opening sections 51, 9 pieces of the adjacent joining-opening sections 50A, and 7 pieces of the adjacent joining-opening sections 50B are arranged in the frame W in FIG. 14.

The sample A contained fine wrinkles in the vertical direction.

### [Sample B]

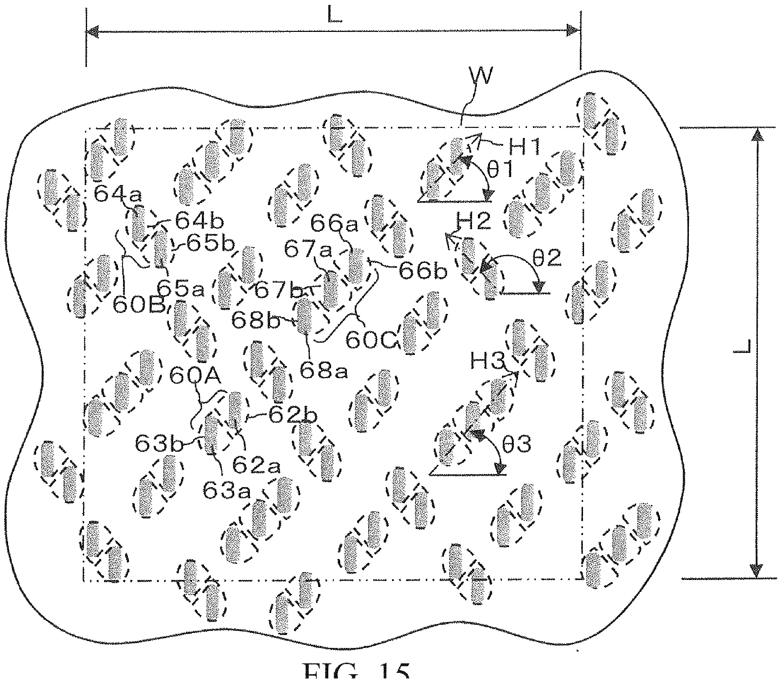

FIG. 15 is a partially enlarged front view of a sample B in a non-stretched state.

As shown in FIG. 15, the sample B are provided with joints and openings similarly to those of the ventral panel 20 (elastic sheet) shown in FIG. 11.

The sample B is provided with joining-opening section 62 constituted by joint 62a and opening 62b, joining-opening section 63 constituted by joint 63a and opening 63b, joining-opening section 64 constituted by joint 64a and opening 64b, joining-opening section 65 constituted by joint 65a and opening 65b, joining-opening section 66 constituted by joint 66a and opening 66b, joining-opening section 67 constituted by joint 67a and opening 67b, and joining-opening section 68 consisting of joint 68a and opening 68b. The joining-opening section 62 and the joining-opening section 63 constitute an adjacent joining-opening section 60A. The joining-opening section 64 and the joining-opening section 65 constitute an adjacent joining-opening section 60B. The joining-opening section 66, the joining-opening section 67 and the joining-opening section 68 constitute an adjacent joining-opening section 60C.

The joints 62a to 68a have oval shapes in which the width in the left-right direction is about 0.3 mm and the length in the vertical direction is about 1.0 mm. The distance d1 between the joint 62a and the joint 63a in the adjacent joining-opening section 60A, the distance d2 between the joint 64a and the joint 65a in the adjacent joining-opening section 60B, and the distance d3 between the joint 66a and the joint 67a and the distance d4 between the joint 67a and the joint 68a in the adjacent joining-opening section 60C are all about 0.5 mm.

The inclination angle θ1 in the first direction H1 in which the joining-opening section 62 and the joining-opening section 63 are arranged in the adjacent joining-opening section 60A is about 50 degrees. The inclination angle Θ2 of the inclination H2 in the second direction in which the joining-opening section 64 and the joining-opening section 65 are arranged in the adjacent joining-opening section 60B is about 130 degrees. The inclination angle θ3 in the third direction H3 in which the joining-opening section 66, the joining-opening section 67 and the joining-opening section 68 are arranged in the adjacent joining-opening section 60C is about 50 degrees.

The sample B is provided with 12 pieces of the adjacent joining-opening sections 60A, 11.5 pieces of the adjacent joining-opening sections 60B and 6 pieces of the adjacent joining-opening sections 60C in the frame W of FIG. 15.

As with the sample A, the sample B contained fine wrinkles in the vertical direction.

### [Sample C]

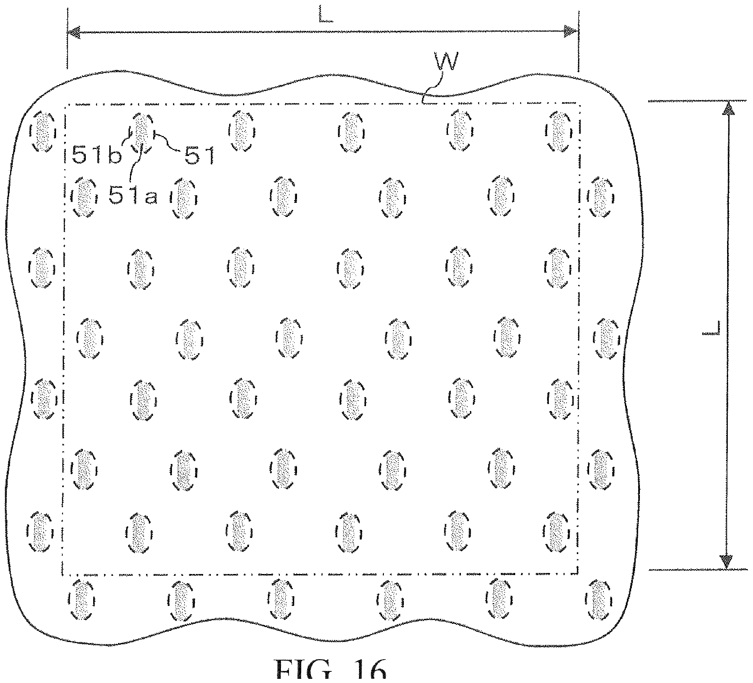

FIG. 16 is a partially enlarged front view of a sample C in a non-stretched state.

As shown in FIG. 16, the sample C is not provided with adjacent joining-opening section and only provided with a joining-opening section 51 constituted by joint 51a and opening 51b. The joint 51a has an oval shape in which the width in the left-right direction is about 0.3 mm and the length in the vertical direction is about 1.0 mm.

In the sample C, joining-opening sections 51 are arranged in a staggered manner. The sample C is provided with a row of left-right direction in which joining-opening sections 51 are arranged at a rate of 5 per unit length L = 10 mm. Seven such left-right direction rows are provided per unit length L = 10 mm in the vertical direction. 35 pieces of the joining-opening sections 51 are arranged in the frame W in FIG. 16.

The sample C contained large wrinkles continuing in the vertical direction.

### [Sample D]

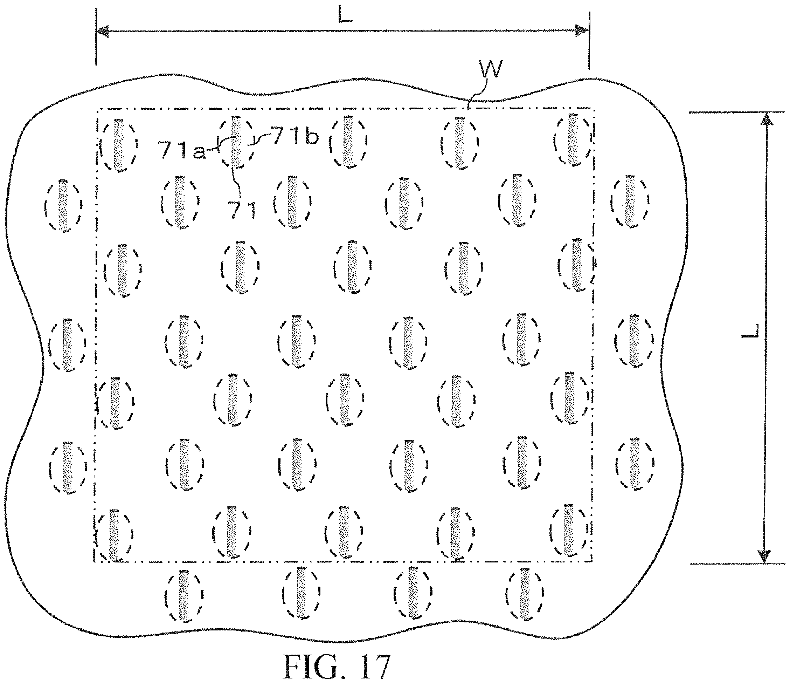

FIG. 17 is a partially enlarged front view of a sample D in a non-stretched state. In the figure, 71 is a joining-opening section, 71a is a joint, and 71b is an opening.

As shown in FIG. 17, similarly to the sample C, the sample D is not provided with adjacent joining-opening section and only provided with a joining-opening section 71 constituted by joint 71a and opening 71b. The joint 71a is larger than the joint 51a and has an oval shape in which the width in the left-right direction is about 0.2 mm and the length in the vertical direction is about 1.5 mm.

In the sample D, joining-opening sections 71 are arranged in a zigzag manner similarly to the sample C. The sample D is provided with a row in the left-right direction in which 5 pieces of the joining-opening sections 71 are arranged per unit length L = 10 mm.

Seven such left-right direction rows are provided per unit length L = 10 mm in the vertical direction. 32 pieces of the joining-opening sections 71 are arranged in the frame W in FIG. 17.

The sample D contained large wrinkles continuing in the vertical direction similarly to the sample C.

### [Test results]

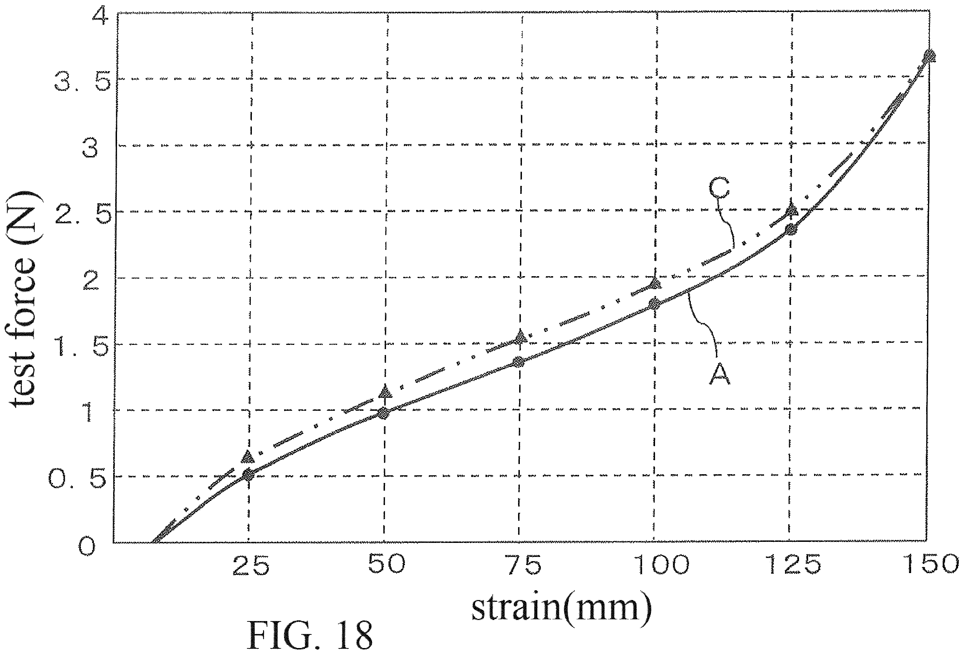

The results of the tensile test on the samples A to D are shown in FIGS. 18 to 21. In each figure, the horizontal axis of the graph represents strain (mm) and the vertical axis represents test force (tensile stress) (N).

FIG. 18 is a graph for a test piece of the sample A and a test piece of the sample C respectively showing a relationship between strain and test force (tensile stress), after a preliminary loading (after extending and restoring the test piece as a preliminary cycle), continuously measured while increasing the strain at a constant speed (300 mm/min) from the state where the test force is 0 (N) to near the elastic limit. A curve A indicated by a solid line shows the relationship between the strain and the test force of the test piece of the sample A, and a curve C indicated by a two-dot chain line shows the relationship between the strain and the test force of the test piece of the sample C.

Sample A had an elastic limit at strain of about 170 mm, and sample C similarly had elastic limit at strain of about 170 mm. From this, the maximum elongation of sample A is about 235% (270 ÷ 115 × 100%) and the maximum elongation of sample C is the same, assuming that the strain of the test piece at the restoring test force 0 (N) is 15 mm.

Table 1 shows the relationship between the main strain and the test force in the graph of FIG. 18.

**[Table 1]**

| strain (mm) | | 25 | 50 | 75 | 100 | 125 | 150 |
|---|---|---|---|---|---|---|---|
| test force (N) | Sample A | 0.5 | 0.98 | 1.37 | 1.78 | 2.36 | 3.67 |
| | Sample C | 0.6 | 1.12 | 1.53 | 1.94 | 2.48 | 3.71 |

In FIG. 18, the strain of the test piece at the test force 0 (N) is not 0 mm. This is because strains remained in the test piece as a result of elongation and restoration (shrinkage) of the test piece as a preliminary cycle performed before the main tensile test, and the strain did not return to 0 mm.

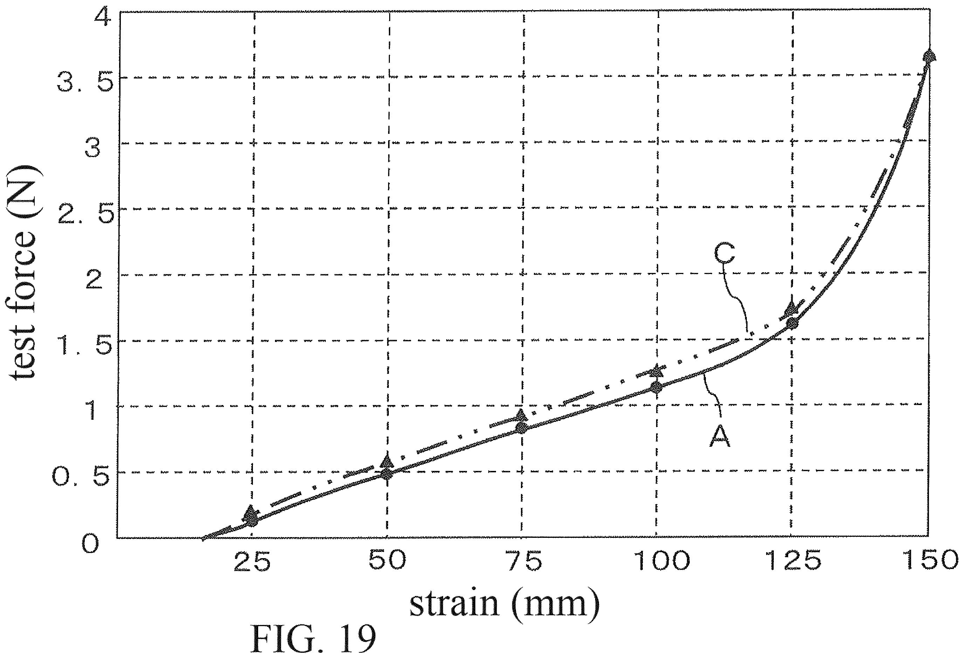

FIG. 19 is a graph for a test piece of the sample A and a test piece of the sample C respectively showing a relationship between strain of the test piece and test force (tensile force), after a preliminary loading, continuously measured while restoring (contracting) the test piece from the state of being displaced to near the elastic limit to 0 (N) of test force at a constant speed (300 mm/min). A curve A indicated by a solid line shows a relation between the strain of the test piece of the sample A and the test force, and a curve C indicated by a two-dot chain line shows the relationship between the strain of the test piece of the sample C and the test force.

Table 2 shows the relationship between the main strain and the test force in the graph of FIG. 19.

**[Table 2]**

| strain (mm) | | 25 | 50 | 75 | 100 | 125 | 150 |
|---|---|---|---|---|---|---|---|
| test force (N) | Sample A | 0.1 | 0.48 | 0.81 | 1.14 | 1.58 | 3.66 |
| | Sample C | 0.16 | 0.58 | 0.94 | 1.28 | 1.72 | 3.69 |

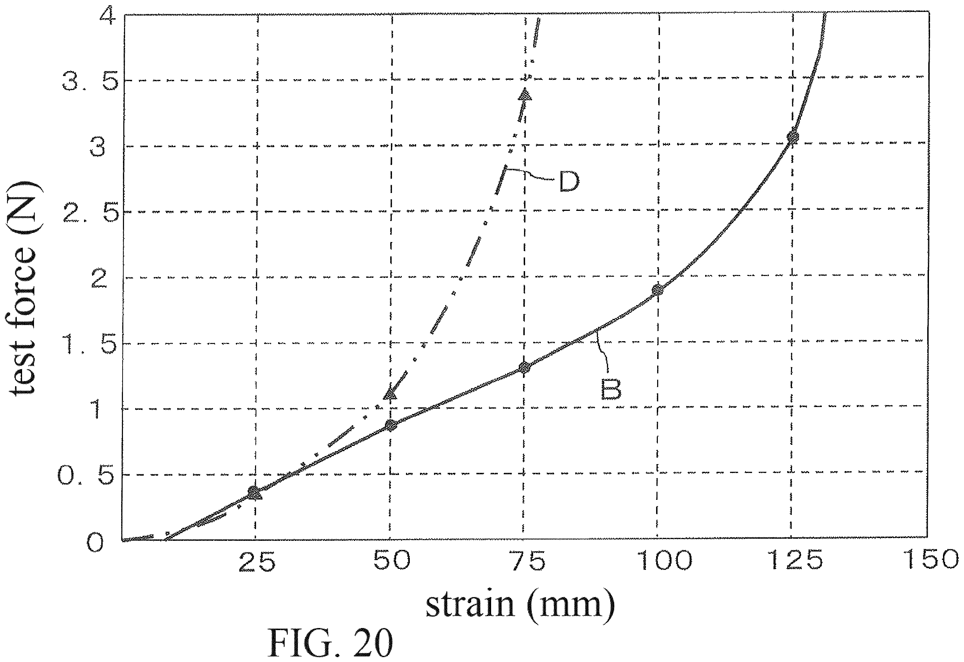

FIG. 20 is a graph for a test piece of the sample B and a test piece of the sample D respectively showing a relationship between strain of the test piece and test force (tensile force), after a preliminary loading, continuously measured while increasing the test force from the 0 (N) state at a constant speed (300 mm/min) and extending the test piece close to the elastic limit. A curve B indicated by a solid line shows a relation between the strain of the test piece of the sample B and the test force, and a curve D indicated by a two-dot chain line shows the relationship between the strain of the test piece of the sample D and the test force.

Sample B had an elastic limit at strain of about 140 mm, and sample D had elastic limit at strain of about 80 mm. From this, the maximum elongation of sample B is about 209% (240 ÷ 115 × 100%) and the maximum elongation of sample D is about 157% (180 ÷ 115 × 100%) the same, assuming that the strain of the test piece at the restoring test force 0 (N) is 15 mm.

Table 3 shows the relationship between the main strain and the test force in the graph of FIG. 20.

**[Table 3]**

| strain (mm) | | 25 | 50 | 75 | 100 | 125 | 150 |
|---|---|---|---|---|---|---|---|
| test force (N) | Sample B | 0.39 | 0.87 | 1.32 | 1.89 | 3.07 | - |
| | Sample D | 0.36 | 1.1 | 3.48 | - | - | - |

FIG. 21 is a graph for a test piece of the sample B and a test piece of the sample D respectively showing a relationship between strain of the test piece and test force (tensile force), after a preliminary loading, continuously measured while restoring (contracting) the test piece from the state of being displaced to near the elastic limit to 0 (N) of test force at a constant speed (300 mm/min). A curve B indicated by a solid line shows a relation between the strain of the test piece of the sample B and the test force, and a curve D indicated by a two-dot chain line shows the relationship between the strain of the test piece of the sample D and the test force.

Table 4 shows the relationship between the main strain and the test force in the graph of FIG. 19.

**[Table 4]**

| strain (mm) | | 25 | 50 | 75 | 100 | 125 | 150 |
|---|---|---|---|---|---|---|---|
| test force (N) | Sample B | 0.06 | 0.38 | 0.72 | 1.15 | 2.3 | - |
| | Sample D | 0.06 | 0.44 | 3.45 | - | - | - |

### [Comparison of the sample A and the sample C]

As shown in FIG. 14, the joining-opening section 51 and the adjacent joining-opening section 50A (or 50B) are alternately arranged in the left-right direction and the vertical direction in the sample A. On the other hand, as shown in FIG. 16, only the joining-opening section 51 is arranged in a staggered manner in the sample C. That is, the sample A is obtained by replacing every other joining-opening section 51 in the sample C with the adjacent joining-opening section 50 A (or 50 B).

The number of the joining-opening section 51 and the adjacent joining-opening section 50 A, 50 B arranged in the frame W is 32 in total in Sample A and 35 in total in Sample C. The densities of the joining-opening section 51 and the adjacent joining-opening sections 50 A, 50 B of the sample A and the density of the joining-opening section 51 of the sample C are approximately the same.

Here, the adjacent joining-opening sections 50A of the sample A are constituted by two joining-opening sections (joining-opening sections 52 and 53) and the adjacent joining-opening sections 50B of the sample A are constituted by two joining-opening sections (joining-opening sections 54 and 55). The sample A has a total of 48 joining-opening sections (joining-opening sections 51 to 55), of which there are 16 joining-opening sections 51, 18 joining-opening sections 52 and 53, and 14 joining-opening sections 54 and 55. The number of joining-opening sections of the sample A is about 1.4 times the number of joining-opening sections 51 of the sample C. The number of opening (opening 51b to 55b) of the sample A is also about 1.4 times the number of opening 51b of the sample C.

Since the sizes of the joints 51a to 55a are the same, the areas of the openings 51b to 55b are also the same, and the area of the opening per unit area of the sample A is about 1.4 times the area of the opening per unit area of the sample C, and then the sample A has better air permeability than the sample C.

Next, as shown in Tables 1 and 2 (FIGS. 18 and 19), the test force (tensile force) of the sample A at the strain of 25 mm to 150 mm is 0.5 to 3.67 N during the elongation and 0.1 to 3.66 N during the restoration (contraction). The test force (tensile force) of the sample C at this strain is 0.6 to 3.71 N during the elongation and 0.16 to 3.69 N during the restoration (contraction). The sample A is 1 to 17% smaller than the sample C. Especially, the test force (tensile force) (in Table 2) at strain of 50 mm to 100 mm during the restoration which is the strain when wearing absorbent article is 0.48 to 1.14 N for the sample A and 0.58 to 1.28 N for the sample C. The sample A is 8 to 17% smaller than the sample C. The extension stress (tensile stress causing constant strain) of the sample A is smaller than the extension stress of the sample C. Also, the maximum elongation of the sample A and that of the sample C are both about 235%.

Therefore, the sample A provided with the adjacent joining-opening sections 50A and 50B has smaller extension stress and is easier to stretch and has better air permeability than the sample C provided with only the joining-opening section 51 and without adjacent joining-opening section. The maximum elongation of the sample A is the same as the maximum elongation of the sample C, and it is constant (200%, for example) or more.

In addition, the sample A contains fine wrinkles in the vertical direction (the direction perpendicular to the stretchable direction), so that it hardly has stiffness and has a soft skin texture. On the other hand, the Sample C contains large wrinkles in the vertical direction, so that it has stiffness and has a hard skin texture.

### [Comparison of the sample B and the sample D]

As shown in FIG. 15, the adjacent joining-opening sections 60A, 60B and 60C are alternately arranged in the left-right direction and the vertical direction in the sample B. On the other hand, as shown in FIG. 17, only the joining-opening sections 71 are arranged in a staggered manner in the sample D.

The number of the adjacent joining-opening sections 60A, 60B, and 60C arranged in the frame W of the sample B is 29.5 in total. The number of joining-opening sections 71 arranged in the frame W of the sample D is 32 in total. The density of the adjacent joining-opening sections 60A, 60B, and 60C of the sample B and the density of the joining-opening section 71 of the sample D are approximately the same.

In this case, the adjacent joining-opening section 60A (60B) of the sample B is constituted by joining-opening sections 62 (64) and 63 (65) which are obtained by dividing the inclined ellipse into two. The major axis of the inclined ellipse and the length of the joining-opening section 71 of the sample D are approximately the same. As a result, the areas of the openings 62b (64b) and 63b (65b) of the adjacent joining-opening section 60A (60B) and the area of the opening 71b of the joining-opening section 71 are approximately the same.

The adjacent joining-opening section 60C is constituted by joining-opening sections 66, 67 and 68 in which the inclined ellipse is divided into three. The major diameter of this inclined ellipse is approximately 1.5 times the length of joining-opening section 71. From this, the area of the opening 66b, 67b and 68b of the adjacent joining-opening section 60C is approximately 1.5 times the area of the opening 71b of the joining-opening section 71.

As a result, the sample B has a total of 23.5 pieces of adjacent joining-opening sections 60A and 60 B and 6 pieces of adjacent joining-opening sections 60C. The area of the opening 62b to 68b corresponds to the area of 32.5 pieces (23.5 pieces + 6 × 1.5 pieces) of opening 71b. Since the area of opening per unit area of the sample B is approximately the same as the opening area per unit area of the sample D, the air permeability of the sample B is not different from the air permeability of the sample D.

Next, as shown in Tables 3 and 4 (FIGS. 20 and 21), the test force (tensile force) of the sample B at the strain of 50 mm to 75 mm is 0.87 to 1.32 N during the elongation and 0.38 to 0.72 N during the restoration (contraction). The test force (tensile force) of the sample D at this strain is 1.1 to 3.48 N during the elongation and 0.44 to 3.45 N during the restoration (contraction). From this, the sample B is smaller than the sample D, and the extension stress of the sample B is smaller than the extension stress of the sample D.

Therefore, the sample B provided with the adjacent joining-opening sections 60A, 60B and 60C has the same air permeability and smaller extension stress and is easier to stretch than the sample D provided with only the joining-opening section 71 and without adjacent joining-opening section. The maximum elongation of the sample B is large and it is constant (200%, for example) or more.

### [Comparison of the sample A and the sample B]

The sample A is provided with the joining-opening section 51 and the adj acent joining-opening sections 50A and 50B constituted by two joining-opening sections. The total number of joining-opening section and adjacent joining-opening section in the frame W is 32 in total. On the other hand, the sample B is provided with the adjacent joining-opening sections 60A and 60B constituted by two joining-opening sections and the adjacent joining-opening section 60C constituted by three joining-opening sections. The total number of the adjacent joining-opening sections in the frame W is 29.5 in total. Thus, the numbers of joining-opening section and adjacent joining-opening section of both samples are almost the same.

This is, the sample B replaces the joining-opening section with the adjacent joining-opening section without changing the densities of joining-opening section and adjacent joining-opening section of sample A, and further increase the joining-opening section of the component in some of the adjacent joining-opening sections.

The number of joining-opening sections 51 to 55 arranged in the frame W in the sample A is 48 in total, of which 16 joining-opening sections 51 and 32 joining-opening sections 52 to 55 are present. The number of openings 51b to 55b is also 48 in total. On the other hand, the number of joining-opening sections 62 to 68 arranged in the frame W of the sample B is 65 in total, of which 47 joining-opening sections 62 to 65 and 18 joining-opening sections 66 to 68 are present. The number of openings 62b to 68b is also 65 in total. Thus, the number of openings 62b to 68b arranged in the frame W of the sample B is about 1.35 times the number of openings 51b to 55b arranged in the same frame of the sample A.

Since the sizes of the joints 51a to 55a of the sample A and the sizes of the joints 62a to 68a of the sample B are the same, the areas of the respective openings 51b to 55b of the sample A and the respective openings 62b to 68b of the sample B are also almost the same. Therefore, the opening area per unit area of sample B is about 1.35 times the area of opening per unit area of sample A, and sample B has better air permeability than sample A.

That is, the sample B changes the number of the adjacent joining-opening sections and the number of the joining-opening sections constituting the adjacent joining-opening section without changing the densities of the joining-opening section and the adjacent joining-opening section of the sample A, and then it has a higher air permeability of the non-stretchable sheet than the sample A

In addition, when comparing the tensile test results of the sample A and the sample B, the test force (tensile force) at a strain of 50 mm to 75 mm which is a strain when wearing an absorbent article is 0.98 to 1.37 N during the elongation and 0.48 to 1.14 N during the restoration (contraction) of the sample A, while this test force (tensile force) is 0.87 to 1.32 N during the elongation and 0.38 to 0.72 N during the restoration (contraction) of the sample B. From this, the sample B is 4 to 21% smaller than the sample A, and the extension stress of the sample B is smaller than the extension stress of the sample A.

Therefore, the elastic sheet provided with the adjacent joining-opening sections can change the number of the adjacent joining-opening sections and the number of the joining-opening sections constituting the adjacent joining-opening section without changing the densities of the joining-opening sections and the adjacent joining-opening sections as in the sample B, and then it is possible to reduce the extension stress when wearing the absorbent article, make it easy to stretch with a small force, and improve the air permeability .

### [Comparison of the sample C and the sample D]

Both the sample C and the sample D are not provided with an adjacent joining-opening section, and joining-opening sections 51, 71 are arranged in a staggered manner in them. 35 joining-opening sections 51 are arranged in the frame W of the sample C, and 32 joining-opening sections 71 are arranged in the frame W of the sample D, and then the densities of joining-opening sections of both samples are approximately the same.

In this case, the joint 71a of the sample D is longer in the vertical direction than the joint 51a of the sample C, and the opening 71b of the sample D is larger in the vertical direction and the left-right direction than the opening 51b of the sample C. Since the length of the vertical direction of joint 71a is 1.5 times the length of the vertical direction of joint 51a, the area of opening 71b is estimated to be about twice the area of opening 51b.

Accordingly, when the area of the opening 71b is about twice the area of the opening 51b, the opening area per unit area of the sample D is about 1.8 times the area of opening per unit area of the sample C. Thus, the sample D has better air permeability of non-stretchable sheet than the sample C.

On the other hand, when comparing the tensile test results of the sample C and the sample D, the maximum elongation of the sample C is about 235%, while the maximum elongation of the sample D is about 157%. The maximum elongation of the sample D is extremely lower than the maximum elongation of the sample C.

Therefore, the elastic sheet provided with only the joining-opening section increases the opening area by increasing the length in the vertical direction of the joint like the sample D. This makes the air permeability better but decreases the maximum elongation extremely.

As described above, the elastic sheet with the adjacent joining-opening section has a small extension stress so that it is easy to stretch the elastic sheet with a small force, and the maximum elongation can be made larger than a certain value and the air permeability can be improved. It also makes it easier to stretch the elastic sheet with a small force by decreasing the extension stress without impairing the air permeability, and it is possible to make the maximum elongation larger than a certain value. Moreover, by forming fine wrinkles, it is possible to reduce the stiffness of the sheet and to create an absorbent article with a good texture.

### INDUSTRIAL APPLICABILITY

The present invention is applicable to an absorbent article using an elastic film, which reduces the extension stress of the absorbent article to make it easy to stretch the absorbent article with a small force, improves the air permeability, increases the maximum elongation of the absorbent article more than a predetermined value, and prevents a large wrinkle continuous in the direction perpendicular to the stretchable direction from occurring and causing stiffness, so as not to impair the softness of the absorbent article, and which can be used for absorbent articles including disposable underwear such as disposable diaper of various forms such as underpants-type, tape fastening type, pad type and the like.

### DESCRIPTION OF THE REFERENCE NUMERAL

1: Underpants-type absorbent article
2: Ventral member
3: Dorsal member
4: Crotch member
5: Waist opening
6 and 7: Leg opening
8 and 9: Side sealed section
20: Ventral panel
20a: Upper edge
20b and 20c: Lower edge
20d: Left end section
20e: Right end section
21: First non-stretchable sheet
21a to 21h: First non-stretchable sheet
22: Second non-stretchable sheet
22a to 22h: Second non-stretchable sheet
23: Elastic film
23a to 23h: Elastic film
30: Dorsal panel
30a: Upper edge
30b and 30c: Lower edge
30d: Left end section
30e: Right end section
31: First non-stretchable sheet
32: Second non-stretchable sheet
33: Elastic film
40: Pad
40b and 40c: Side edge
41: Absorber
42: Outer sheet
42a: Polyethylene film
42b: Nonwoven fabric
43: Inner sheet
44 and 45: Gather sheet
44a and 45a: End section
44b and 45b: Standing section
51 to 55: Joining-opening section
51a to 55a: Joint
51b to 55b: Opening
50A and 50B: Adjacent joining-opening section
62 to 68: Joining-opening section
62a to 68a: Joint
62b to 68b: Opening
60A, 60B and 60C: Adjacent joining-opening section
71: Joining-opening section
71a: Joint
71b: Opening
A to D: Curve
d1, d2 and d3: Interval
E1, E2, E3, and E4: Elastic member
G1 to G5: Dividing line
H1: First direction
H2: Second direction
H3: Third direction
W: Frame

## Claims

1. An absorbent article (1) comprising:
a stretchable region, wherein
the stretchable region of the absorbent article (1) is made of an elastic sheet in which a first non-stretchable sheet (21, 31), an elastic film (23, 33) and a second non-stretchable sheet (22, 32) are laminated in this order,
the elastic sheet is provided with a plurality of joining-opening sections (51 to 55, 62 to 68) that are constituted by joints (51a to 55a, 62a to 68a) and openings (51b to 55b, 62b to 68b),
the joints (51a to 55a, 62a to 68a) are formed by welding the first non-stretchable sheet (21, 31) and the second non-stretchable sheet (22, 32) in a state where the elastic film (23, 33) is stretched in a stretchable direction of the stretchable region,
a length of the joint (51a to 55a, 62a to 68a) in the stretchable direction is shorter than a length of the joint (51a to 55a, 62a to 68a) in a direction perpendicular to the stretchable direction,
the length of the joint (51a to 55a, 62a to 68a) in the stretchable direction is 0.1 to 1.0 mm,
the length of the joint (51a to 55a, 62a to 68a) in the direction perpendicular to the stretchable direction is 0.3 to 3.0 mm,
the openings (51b to 55b, 62b to 68b) are formed in the elastic film (23, 33) by forming the joints (51a to 55a, 62a to 68a),
some or all of the joining-opening sections (52 to 55, 62 to 68) form adjacent joining-opening sections (50A, 50B, 60A, 60B, 60C) constituted by two or three of the joining-opening sections (52 to 55, 62 to 68) which are close to each other,
the distance (d1, d2, d3, d4) between the joints (52a to 55a, 62a to 68a) of the adjacent joining-opening sections (50A, 50B, 60A, 60B, 60C) in the stretchable direction is 0.3 to 1.5 mm,
the openings (52b to 55b, 62b to 68b) of the adjacent joining-opening sections (50A, 50B, 60A, 60B, 60C) are ununited each other,
the joining-opening sections (52 to 55, 62 to 68) in the adjacent joining-opening section (50A, 50B, 60A, 60B, 60C) are arranged adjacently in a direction inclined with respect to the stretchable direction, and
the adjacent joining-opening sections (50A, 50B, 60A, 60B, 60C) are arranged such that dividing lines (G1-G5) to divide the adjacent joining-opening section(50A, 50B, 60A, 60B, 60C) into two in a direction perpendicular to the direction (H1-H3) inclined with respect to the stretchable direction are not aligned on a straight line.

2. The absorbent article (1) according to claim 1 , wherein
the adjacent joining-opening section (50A, 50B) and a single joining-opening section (51) not forming the adjacent joining-opening section is alternately arranged in the stretchable direction.

3. The absorbent article (1) according to claim 1 , wherein
the joining-opening sections (52, 53, 62, 63) constituting the adjacent joining-opening section (50A, 60A) are arranged in a first direction (H1) which is inclined with respect to the stretchable direction,
the other joining-opening sections (54, 55, 64, 65) constituting the other adjacent joining-opening section (50B, 60B) are also arranged in a second direction (H2) which is inclined with respect to the stretchable direction and is different from the first direction (H1)
the adjacent joining-opening sections (50A, 60A) are arranged such that dividing lines to divide the adjacent joining-opening section (50A, 60A) into two in a direction perpendicular to the first direction (H1) are not aligned on a straight line, and
the other adjacent joining-opening sections (50B, 60B) are arranged such that dividing lines to divide the other adjacent joining-opening section (50B, 60B) into two in a direction perpendicular to the second direction (H2) are not aligned on a straight line

4. The absorbent article (1) according to claim 3, wherein
the second direction (H2) is a direction in which the first direction (H1) is inverted with a direction perpendicular to the stretchable direction as an axis.

5. The absorbent article (1) according to claim 4, wherein
the adjacent joining-opening sections (50A, 60A) constituted by the joining-opening sections (52, 53, 62, 63) arranged in the first direction (H1) and the other adjacent joining-opening sections (50B, 60B) constituted by the other joining-opening sections (54, 55, 64, 65) arranged in the second direction (H2) are evenly arranged.

## Patentansprüche

1. Saugfähiger Artikel (1) umfassend:
einen dehnbaren Bereich, wobei
der dehnbare Bereich des saugfähigen Artikels (1) aus einer elastischen Lage hergestellt ist, in der eine erste nicht dehnbare Lage (21, 31), eine elastische Folie (23, 33) und eine zweite nicht dehnbare Lage (22, 32) in dieser Reihenfolge geschichtet sind,
die elastische Lage mit einer Vielzahl von Verbindungsöffnungsabschnitten (51 bis 55, 62 bis 68) versehen ist, die durch Verbindungen (51a bis 55a, 62a bis 68a) und Öffnungen (51b bis 55b, 62b bis 68b) gebildet werden,
die Verbindungen (51a bis 55a, 62a bis 68a) durch Verschweißen der ersten nicht dehnbaren Lage (21, 31) und der zweiten nicht dehnbaren Lage (22, 32) in einem Zustand gebildet werden, in dem die elastische Folie (23, 33) in einer Dehnungsrichtung des dehnbaren Bereichs gedehnt ist,
eine Länge der Verbindung (51a bis 55a, 62a bis 68a) in der Dehnungsrichtung kürzer ist als eine Länge der Verbindung (51a bis 55a, 62a bis 68a) in einer Richtung senkrecht zu der Dehnungsrichtung,
die Länge der Verbindung (51a bis 55a, 62a bis 68a) in der Dehnungsrichtung 0,1 bis 1,0 mm beträgt,
die Länge der Verbindung (51a bis 55a, 62a bis 68a) in der Richtung senkrecht zur Dehnungsrichtung 0,3 bis 3,0 mm beträgt,
die Öffnungen (51b bis 55b, 62b bis 68b) in der elastischen Folie (23, 33) durch Bildung der Verbindungen (51a bis 55a, 62a bis 68a) gebildet werden,
einige oder alle der Verbindungsöffnungsabschnitte (52 bis 55, 62 bis 68) benachbarte Verbindungsöffnungsabschnitte (50A, 50B, 60A, 60B, 60C) bilden, die aus zwei oder drei der Verbindungsöffnungsabschnitte (52 bis 55, 62 bis 68) bestehen, die nahe beieinander liegen,
der Abstand (d1, d2, d3, d4) zwischen den Verbindungen (52a bis 55a, 62a bis 68a) der benachbarten Verbindungsöffnungsabschnitte (50A, 50B, 60A, 60B, 60C) in der Dehnungsrichtung 0,3 bis 1,5 mm beträgt,
die Öffnungen (52b bis 55b, 62b bis 68b) der benachbarten Verbindungsöffnungsabschnitte (50A, 50B, 60A, 60B, 60C) nicht miteinander verbunden sind,
die Verbindungsöffnungsabschnitte (52 bis 55, 62 bis 68) in dem benachbarten Verbindungsöffnungsabschnitt (50A, 50B, 60A, 60B, 60C) in einer zur Dehnungsrichtung geneigten Richtung benachbart angeordnet sind, und
die benachbarten Verbindungsöffnungsabschnitte (50A, 50B, 60A, 60B, 60C) so angeordnet sind, dass Teilungslinien (G1-G5) zum Teilen des benachbarten Verbindungsöffnungsabschnitts (50A, 50B, 60A, 60B, 60C) in zwei in einer Richtung senkrecht zu der Richtung (H1-H3) geneigten Richtung in Bezug auf die Dehnungsrichtung nicht auf einer geraden Linie ausgerichtet sind.

2. Saugfähiger Artikel (1) nach Anspruch 1, wobei
der benachbarte Verbindungsöffnungsabschnitt (50A, 50B) und ein einzelner Verbindungsöffnungsabschnitt (51), der nicht den benachbarten Verbindungsöffnungsabschnitt bildet, in der Dehnungsrichtung abwechselnd angeordnet sind.

3. Saugfähiger Artikel (1) nach Anspruch 1, wobei
die Verbindungsöffnungsabschnitte (52, 53, 62, 63), die den benachbarten Verbindungsöffnungsabschnitt (50A, 60A) bilden, in einer ersten Richtung (H1) angeordnet sind, die in Bezug auf die Dehnungsrichtung geneigt ist,
die anderen Verbindungsöffnungsabschnitte (54, 55, 64, 65), die den anderen benachbarten Verbindungsöffnungsabschnitt (50B, 60B) bilden, ebenfalls in einer zweiten Richtung (H2) angeordnet sind, die in Bezug auf die Dehnungsrichtung geneigt ist und sich von der ersten Richtung (H1) unterscheidet
die benachbarten Verbindungsöffnungsabschnitte (50A, 60A) so angeordnet sind, dass Teilungslinien zur Unterteilung des benachbarten Verbindungsöffnungsabschnitts (50A, 60A) in zwei in einer Richtung senkrecht zur ersten Richtung (H1) nicht auf einer geraden Linie ausgerichtet sind, und
die anderen benachbarten Verbindungsöffnungsabschnitte (50B, 60B) so angeordnet sind, dass Teilungslinien zur Unterteilung des anderen benachbarten Verbindungsöffnungsabschnitts (50B, 60B) in zwei in einer Richtung senkrecht zu der zweiten Richtung (H2) nicht auf einer geraden Linie ausgerichtet sind

4. Saugfähiger Artikel (1) nach Anspruch 3, wobei
die zweite Richtung (H2) eine Richtung ist, in der die erste Richtung (H1) mit einer Richtung senkrecht zu der Dehnungsrichtung als Achse invertiert ist.

5. Saugfähiger Artikel (1) nach Anspruch 4, wobei
die benachbarten Verbindungsöffnungsabschnitte (50A, 60A), die durch die in der ersten Richtung (H1) angeordneten Verbindungsöffnungsabschnitte (52, 53, 62, 63) gebildet werden, und die anderen benachbarten Verbindungsöffnungsabschnitte (50B, 60B), die durch die in der zweiten Richtung (H2) angeordneten anderen Verbindungsöffnungsabschnitte (54, 55, 64, 65) gebildet werden, gleichmäßig angeordnet sind.

## Revendications

1. Article absorbant (1) comprenant :
une région extensible, dans lequel :
la région extensible de l'article absorbant (1) est réalisée avec une feuille élastique dans laquelle une première feuille non extensible (21, 31), un film élastique (23, 33) et une seconde feuille non extensible (22, 32) sont stratifiés dans cet ordre,
la feuille élastique est prévue avec une pluralité de sections de joint-ouverture (51 à 55, 62 à 68) qui sont constituées par des joints (51a à 55a, 62a à 68a) et des ouvertures (51b à 55b, 62b à 68b),
les joints (51a à 55a, 62a à 68a) sont formés en soudant la première feuille non extensible (21, 31) et la seconde feuille non extensible (22, 32) dans un état dans lequel le film élastique (23, 33) est étiré dans une direction extensible de la région extensible,
une longueur du joint (51a à 55a, 62a à 68a) dans la direction extensible, est plus courte qu'une longueur du joint (51a à 55a, 62a à 68a) dans une direction perpendiculaire à la direction extensible,
la longueur du joint (51a à 55a, 62a à 68a) dans la direction extensible est de 0,1 à 1,0 mm,
la longueur du joint (51a à 55a, 62a à 68a) dans la direction perpendiculaire à la direction extensible est de 0,3 à 3,0 mm,
les ouvertures (51b à 55b, 62b à 68b) sont formées dans le film élastique (23, 33) en formant les joints (51a à 55a, 62a à 68a),
certaines ou la totalité des sections de joint-ouverture (52 à 55, 62 à 68) forment des sections de joint-ouverture (50A, 50B, 60A, 60B, 60C) constituées par deux ou trois des sections de joint-ouverture (52 à 55, 62 à 68) qui sont proches les unes des autres,
la distance (d1, d2, d3, d4) entre les joints (52a à 55a, 62a à 68a) des sections de joint-ouverture (50A, 50B, 60A, 60B, 60C) dans la direction extensible est de 0,3 à 1,5 mm,
les ouvertures (52b à 55b, 62b à 68b) des sections de joint-ouverture (50A, 50B, 60A, 60B, 60C) sont désunies les unes par rapport aux autres,
les sections de joint-ouverture (52 à 55, 62 à 68) dans la section de joint-ouverture (50A, 50B, 60A, 60B, 60C) adjacente sont agencées de manière adjacente dans une direction inclinée par rapport à la direction extensible, et
les sections de joint-ouverture (50A, 50B, 60A, 60B, 60C) adjacentes sont agencées de sorte que les lignes de division (G1-G5) pour diviser la section de joint-ouverture (50A, 50B, 60A, 60B, 60C) adjacente en deux dans une direction perpendiculaire à la direction (H1-H3) inclinée par rapport à la direction extensible ne sont pas alignées sur une ligne droite.

2. Article absorbant (1) selon la revendication 1, dans lequel :
la section de joint-ouverture (50A, 50B) adjacente et la section de joint-ouverture (51) unique ne formant pas la section de joint-ouverture adjacente sont agencées de manière alternée dans la direction extensible.

3. Article absorbant (1) selon la revendication 1, dans lequel :
les sections de joint-ouverture (52, 53, 62, 63) constituant la section de joint-ouverture (50A, 60A) adjacente sont agencées dans une première direction (H1) qui est inclinée par rapport à la direction extensible,
les autres sections de joint-ouverture (54, 55, 64, 65) constituant l'autre section de joint-ouverture (50B, 60B) adjacente sont également agencées dans une seconde direction (H2) qui est inclinée par rapport à la direction extensible et est différente de la première direction (H1),
les sections de joint-ouverture (50A, 60A) adjacentes sont agencées de sorte que les lignes de division pour diviser la section de joint-ouverture (50A, 60A) adjacente en deux dans une direction perpendiculaire à la première direction (H1) ne sont pas alignées sur une ligne droite, et
les autres sections de joint-ouverture (50B, 60B) adjacentes sont agencées de sorte que les lignes de division pour diviser l'autre section de joint-ouverture (50B, 60B) adjacente en deux dans une direction perpendiculaire à la seconde direction (H2) ne sont pas alignées sur une ligne droite .

4. Article absorbant (1) selon la revendication 3, dans lequel :
la seconde direction (H2) est une direction dans laquelle la première direction (H1) est inversée par rapport à une direction perpendiculaire à la direction extensible en tant qu'axe.

5. Article absorbant (1) selon la revendication 4, dans lequel :
les sections de joint-ouverture (50A, 60A) adjacentes constituées par les sections de joint-ouverture (52, 53, 62, 63) agencées dans la première direction (H1) et les autres sections de joint-ouverture (50B, 60B) adjacentes constituées par les autres sections de joint-ouverture (54, 55, 64, 65) agencées dans la seconde direction (H2) sont agencées de manière uniforme.
